(19) Europäisches Patentamt
European Patent Office
Office européen des brevets

(11) Veröffentlichungsnummer: **0 306 698 B1**

(12) **EUROPÄISCHE PATENTSCHRIFT**

(45) Veröffentlichungstag der Patentschrift: **25.03.92**

(21) Anmeldenummer: **88112443.2**

(22) Anmeldetag: **01.08.88**

(51) Int. Cl.[5]: **C07D 401/06**, C07D 403/06, C07D 413/06, C07D 417/06, C07D 487/04, C07D 471/04, C07D 495/04, C07D 495/14, C07D 471/14, C07D 487/14, A61K 31/55, //(C07D487/04, 243:00,209:00),(C07D487/04, 243:00,231:00)

(54) **Neue kondensierte Diazepinone, Verfahren zu ihrer Herstellung und diese Verbindungen enthaltende Arzneimittel.**

(30) Priorität: **13.08.87 DE 3726908**

(43) Veröffentlichungstag der Anmeldung:
**15.03.89 Patentblatt 89/11**

(45) Bekanntmachung des Hinweises auf die Patenterteilung:
**25.03.92 Patentblatt 92/13**

(84) Benannte Vertragsstaaten:
**AT BE CH DE ES FR GB GR IT LI LU NL SE**

(73) Patentinhaber: **Dr. Karl Thomae GmbH**
**Postfach 1755**
**W-7950 Biberach 1(DE)**

(72) Erfinder: **Engel, Wolfhard, Dr. Dipl.-Chem.**
**Mozartstrasse 13**
**W-7950 Biberach 1(DE)**
Erfinder: **Trummlitz, Günter, Dr. Dipl.-Chem.**
**Buchenweg 27**
**W-7951 Warthausen(DE)**
Erfinder: **Eberlein, Wolfgang, Dr. Dipl.-Chem.**
**Obere Au 6**
**W-7950 Biberach 1(DE)**
Erfinder: **Mihm, Gerhard, Dr. Dipl.-Chem.**
**Nickeleshalde 5/1**
**W-7950 Biberach 1(DE)**

EP 0 306 698 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(56) Entgegenhaltungen:
EP-A- 0 023 707
EP-A- 0 039 519
EP-A- 0 085 892
EP-A- 0 085 899
EP-A- 0 086 982
EP-A- 0 125 607
EP-A- 0 139 627
EP-A- 0 156 191
EP-A- 0 213 450
EP-A- 0 254 955
DE-A- 2 724 434
DE-A- 2 724 478
US-A- 3 660 380

Erfinder: **Mayer, Norbert, Dr.**
**Friedrich-Ebert-Strasse 66**
**W-7950 Biberach 1(DE)**
Erfinder: **De Jonge, Adriaan, Dr.**
**De Boomgaard 19**
**NL-3971 LD Driebergen(NL)**
Erfinder: **Rudolf, Klaus, Dr.**
**Marktplatz 38**
**W-7950 Biberach 1(DE)**

## Beschreibung

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 ist beschrieben, daß durch Einführung neuartiger Aminoacylreste gegenüber den Verbindungen der obengenannten Publikationen völlig andersartige wertvolle pharmakologische Eigenschaften induziert werden können. Gegenüber diesen Verbindungen zeichnen sich die erfindungsgemäßen kondensierten Diazepinone bei vergleichbarer oder verbesserter Selektivität und Resorption nach oraler Gabe durch eine wesentlich verstärkte Wirkung und ausgeprägte Hydrolysestabilität aus.

Die neuen kondensierten Diazepinone besitzen die allgemeine Formel I,

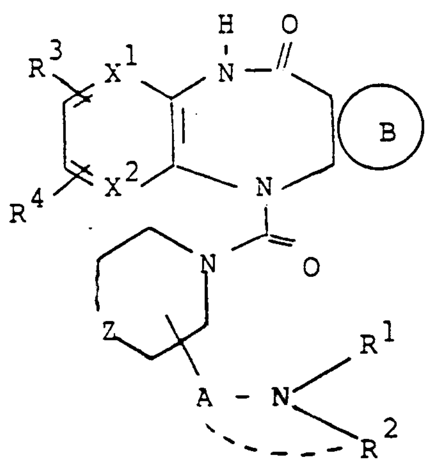

(I)

in der

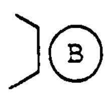

einen der zweiwertigen Reste

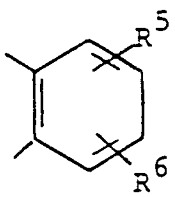 , 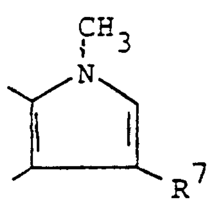 , 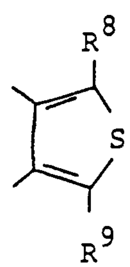 , 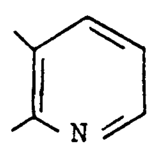 oder 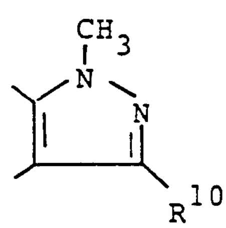

(S) (T) (U) (V) (W)

darstellt und

$X^1$, $X^2$, A, $R^1$ bis $R^{10}$ und Z die folgenden Bedeutungen besitzen:

$X^1$ und $X^2$ stellen eine =CH-Gruppe dar oder, sofern

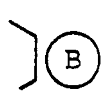

die Bedeutungen der oben genannten, zweiwertigen Reste (S), (U) oder (W) annimmt, können beide oder nur $X^1$ oder nur $X^2$ auch ein Stickstoffatom darstellen;
A ist ein geradkettiger oder verzweigter gesättigter Alkylenrest mit zwei bis sieben Kohlenstoffatomen; der auch durch ein Sauerstoff- oder Schwefelatom oder durch die Methylimino- oder Ethyliminogruppe unterbrochen sein kann;
Z eine Einfachbindung, ein Sauerstoff- oder Schwefelatom, die Methylen- oder 1,2-Ethylengruppe;
$R^1$ ein verzweigter oder unverzweigter Alkylrest mit 1 bis 4 Kohlenstoffatomen oder die Benzylgruppe;
$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;
$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die $N\text{-}CH_3$-Gruppe unterbrochen sein kann;
$R^2$ kann aber auch mit A über eine Alkylenbrücke in der Weise verknüpft sein, daß zusammen mit der Gruppe $NR^1$ ein gesättigtes, 5-, 6- oder 7-gliedriges heterocyclisches Ringsystem entsteht;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Wasserstoffatom, ein Fluor-, Chlor- oder Bromatom oder eine Alkylgruppe mit 1 bis 4 C-Atomen;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe.

Für den Fall daß

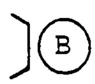

den zweiwertigen Rest (T) darstellt und $R^7$ ein Wasserstoffatom ist, können $R^3$ kein Chloratom und Z kein Schwefelatom bedeuten.

Bevorzugte Verbindungen der obigen allgemeinen Formel I sind solche, in denen <u>entweder</u>
$X^1$ eine =CH-Gruppe,
$X^2$ ein Stickstoffatom und

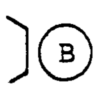

den zweiwertigen Rest (S) darstellt, mit der Maßgabe, daß $R^3$, $R^4$ und $R^5$ Wasserstoffatome sind und $R^6$ ein Wasserstoffatom, ein Chlor-oder Bromatom oder eine Methyl- oder Ethylgruppe in 8- oder 9-Stellung des Heterocyclus ist,
<u>oder</u> $X^1$ und $X^2$ =CH-Gruppen darstellen und

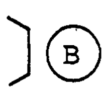

die Bedeutung des zweiwertiges Restes (U) annimmt, wobei $R^8$ ein Wasserstoffatom und $R^9$ die Methylgruppe ist, oder den Rest (V) darstellt, wobei mindestens einer der Reste $R^3$ und $R^4$ ein Wasserstoffatom ist,
A eine zwei- bis viergliedrige Alkylenkette in 3- oder 4-Stellung des gesättigten heterocyclischen Ringes,
Z die Methylengruppe und
$R^1$ und $R^2$ Alkylgruppen mit 1 bis 4 Kohlenstoffatomen oder zusammen mit dem eingeschlossenen Stickstoffatom den 1-Pyrrolidinyl-, 1-Piperidinyl-, 4-Morpholinyl- oder Hexahydro-1H-1-azepinyl-Rest bedeuten.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:
6,11-Dihydro-11-[[3-[3-(1-piperidinyl)propyl]-1-piperidinyl]carbonyl]-5H-pyrido[2,3-b][1,5]benzodiazepin-5-on
11-[[3-[2-(Cyclopentylmethylamino)ethyl]-1-piperidinyl]carbonyl]-6,11-dihydro-5H-pyrido[2,3-b][1,5]-benzodiazepin-5-on
6,11-Dihydro-11-[[3-[3-(1-pyrrolidinyl)propyl]-1-piperidinyl]carbonyl]-5H-pyrido[2,3-b][1,5]benzodiazepin-5-on
6,11-Dihydro-11-[[3-[2-(hexahydro-1H-1-azepinyl)ethyl]-1-piperidinyl]carbonyl]-5H-pyrido[2,3-b][1,5]-benzodiazepin-5-on-methansulfonat
D,L-6,11-Dihydro-11-[[3-[2-(1-piperidinyl)ethyl]-1-piperidinyl]carbonyl]-5H-pyrido[2,3-b][1,5]benzodiazepin-5-on
(+)-6,11-Dihydro-11-[[3-[2-(1-piperidinyl)ethyl]-1-piperidinyl]carbonyl]-5H-pyrido[2,3-b][1,5]benzodiazepin-5-on
(-)-6,11-Dihydro-11-[[3-[2-(1-piperidinyl)ethyl]-1-piperidinyl]carbonyl]-5H-pyrido[2,3-b][1,5]benzodiazepin-5-on
11-[[3-[2-(Diethylamino)ethyl]-1-piperidinyl]carbonyl]-6,11-dihydro-5H-pyrido[2,3-b][1,5]benzodiazepin-5-on
11-[[3-[3-(Diethylamino)propyl]-1-piperidinyl]carbonyl]-6,11-dihydro-5H-pyrido[2,3-b][1,5]benzodiazepin-5-on
6,11-Dihydro-11-[[4-[4-(1-pyrrolidinyl)butyl]-1-piperidinyl]carbonyl]-5H-pyrido[2,3-b][1,5]benzodiazepin-5-on-hydrochlorid
6,11-Dihydro-11-[[3-[3-(4-morpholinyl)propyl]-1-piperidinyl]carbonyl]-5H-pyrido[2,3-b][1,5]benzodiazepin-5-on
11-[[2-[[[3-(Diethylamino)propyl]methylamino]methyl]-1-piperidinyl]carbonyl]-6,11-dihydro-5H-pyrido[2,3-b]-[1,5]benzodiazepin-5-on-dihydrochlorid
11-[[2-[2-[[3-(Diethylamino)propyl]methylamino]ethyl]-1-piperidinyl]carbonyl]-6,11-dihydro-5H-pyrido[2,3-b]-[1,5]benzodiazepin-5-on
6,11-Dihydro-11-[[3-[3-(1-piperidinyl)propyl]-1-pyrrolidinyl]carbonyl]-5H-pyrido[2,3-b][1,5]benzodiazepin-5-on
6,11-Dihydro-11-[[3-[3-(1-piperidinyl)propyl]-hexahydro-1H-1-azepinyl]carbonyl]-5H-pyrido[2,3-b][1,5]-benzodiazepin-5-on
11-[[3-[2-[(Cyclopentyl)ethylamino]ethyl]-1-pyrrolidinyl]carbonyl]-6,11-dihydro-5H-pyrido[2,3-b][1,5]-benzodiazepin-5-on
11-[[3-[2-[(Cyclobutylmethyl)methylamino]ethyl]-1-pyrrolidinyl]carbonyl]-6,11-dihydro-5H-pyrido[2,3-b][1,5]-benzodiazepin-5-on
6,11-Dihydro-11-[[3-[4-(1-pyrrolidinyl)butyl]-1-pyrrolidinyl]carbonyl]-5H-pyrido[2,3-b][1,5]benzodiazepin-5-on
6,11-Dihydro-11-[[3-[2-(1-pyrrolidinyl)ethyl]-hexahydro-1H-1-azepinyl]carbonyl]-5H-pyrido[2,3-b][1,5]-benzodiazepin-5-on
6,11-Dihydro-11-[[3-[2-(hexahydro-1H-1-azepinyl)ethyl]-1-pyrrolidinyl]carbonyl]-5H-pyrido[2,3-b][1,5]-benzodiazepin-5-on
6,11-Dihydro-11-[[3-[2-(hexahydro-1H-1-azepinyl]ethyl]-hexahydro-1H-1-azepinyl]carbonyl]-5H-pyrido[2,3-b]-[1,5-benzodiazepin-5-on
11-[[3-[2-(Diethylamino)ethyl]-1-pyrrolidinyl]carbonyl]-6,11-dihydro-5H-pyrido[2,3-b][1,5]benzodiazepin-5-on
11-[[3-[2-(Diethylamino)ethyl]hexahydro-1H-1-azepinyl]carbonyl]-6,11-dihydro-5H-pyrido[2,3-b][1,5]-benzodiazepin-5-on
6,11-Dihydro-11-[[3-[4-(dimethylamino)butyl]-1-piperidinyl]carbonyl]-5H-pyrido[2,3-b][1,5]benzodiazepin-5-on
11-[[3-[2-(Butylethylamino)ethyl]-1-piperidinyl]carbonyl]6,11-dihydro-5H-pyrido[2,3-b][1,5]benzodiazepin-5-on
6,11-Dihydro-11-[[3-[3-(dipropylamino)propyl]-1-piperidinyl]carbonyl]-5H-pyrido[2,3-b][1,5]benzodiazepin-5-on
11-[[3-[2-(Dibutylamino)ethyl]-1-piperidinyl]carbonyl]-6,11-dihydro-5H-pyrido[2,3-b][1,5]benzodiazepin-5-on
6,11-Dihydro-11-[[3-[ [(1-methyl-4-piperidinyl)methylamino]methyl]-1-piperidinyl]carbonyl]-5H-pyrido[2,3-b]-[1,5]benzodiazepin-5-on
6,11-Dihydro-11-[[2-[[(1-methyl-4-piperidinyl)methylamino]methyl]-4-morpholinyl]carbonyl]-5H-pyrido[2,3-b]-[1,5]benzodiazepin-5-on
6,11-Dihydro-11-[[4-[3-(1-piperidinyl)propyl]-1-piperidinyl]carbonyl]-5H-pyrido[2,3-b][1,5]benzodiazepin-5-on
11-[[4-[3-(Diethylamino)propyl]-1-piperidinyl]carbonyl]-6,11-dihydro-5H-pyrido[2,3-b][1,5]benzodiazepin-5-on
11-[[4-[4-(Diethylamino)butyl]-1-piperidinyl]carbonyl]-6,11-dihydro-5H-pyrido[2,3-b][1,5]benzodiazepin-5-on
5,11-Dihydro-11-[[4-[3-(1-piperidinyl)propyl]-1-piperidinyl]carbonyl]-6H-pyrido[2,3-b][1,4]benzodiazepin-6-on

5,11-Dihydro-11-[[3-[3-(1-piperidinyl)propyl]-1-piperidinyl]carbonyl]-6H-pyrido[2,3-b][1,4]benzodiazepin-6-on
5,11-Dihydro-11-[[4-[4-(1-piperidinyl)butyl]-1-piperidinyl]carbonyl]-6H-pyrido[2,3-b][1,4]benzodiazepin-6-on
11-[[3-[2-[(Cycylopentyl)methylamino]ethyl]-1-piperidinyl]carbonyl]-5,11-dihydro-6H-pyrido[2,3-b][1,4]-benzodiazepin-6-on
5,11-Dihydro-11-[[3-[3-(1-pyrrolidinyl)propyl]-1-piperidinyl]carbonyl]-6H-pyrido[2,3-b][1,4]benzodiazepin-6-on
5,11-Dihydro-11-[[4-[4-(1-pyrrolidinyl)butyl]-1-piperidinyl]carbonyl]-6H-pyrido[2,3-b][1,4]benzodiazepin-6-on
5,11-Dihydro-11-[[3-[4-(1-pyrrolidinyl)butyl]-1-piperidinyl]carbonyl]-6H-pyrido[2,3-b][1,4]benzodiazepin-6-on
5,11-Dihydro-11-[[3-[3-(1-pyrrolidinyl)propyl]-hexahydro-1H-1-azepinyl]carbonyl]-6H-pyrido[2,3-b][1,4]-benzodiazepin-6-on
(+)-5,11-Dihydro-11-[[3-[2-(1-piperidinyl)ethyl]-1-piperidinyl]carbonyl]-6H-pyrido[2,3-b][1,4]benzodiazepin-6-on
(-)-5,11-Dihydro-11-[[3-[2-(1-piperidinyl)ethyl]-1-piperidinyl]carbonyl]-6H-pyrido[2,3-b][1,4]benzodiazepin-6-on
5,11-Dihydro-11-[[3-[2-(hexahydro-1H-1-azepinyl)ethyl]-1-piperidinyl]carbonyl-6H-pyrido[2,3-b][1,4]-benzodiazepin-6-on-methansulfonat
11-[[3-[2-(Diethylamino)ethyl]-1-piperidinyl]carbonyl]-5,11-dihydro-6H-pyrido[2,3-b][1,4]benzodiazepin-6-on
11-[[3-[3-(Diethylamino)propyl]-1-piperidinyl]carbonyl]-5,11-dihydro-6H-pyrido[2,3-b][1,4]benzodiazepin-6-on
11-[[2-[3-(Diethylamino)propyl]-4-morpholinyl]carbonyl]-5,11-dihydro-6H-pyrido[2,3-b][1,4]benzodiazepin-6-on
11-[[4-[3-(Diethylamino)propyl]-1-piperidinyl]carbonyl]-5,11-dihydro-6H-pyrido[2,3-b][1,4]benzodiazepin-6-on
8-Chlor-5,11-dihydro-11-[[3-[2-(1-piperidinyl)ethyl]-1-piperidinyl]carbonyl]-6H-pyrido[2,3-b][1,4]-benzodiazepin-6-on
9-Chlor-5,11-dihydro-11-[[3-[2-(1-piperidinyl)ethyl]-1-piperidinyl]carbonyl]-6H-pyrido[2,3-b][1,4]-benzodiazepin-6-on
5,11-Dihydro-8-methyl-11-[[3-[2-(1-piperidinyl)ethyl]-1-piperidinyl]carbonyl]-6H-pyrido[2,3-b][1,4]-benzodiazepin-6-on
5,11-Dihydro-8-ethyl-11-[[3-[2-(1-piperidinyl)ethyl]-1-piperidinyl]carbonyl]-6H-pyrido[2,3-b][1,4]-benzodiazepin-6-on
5,11-Dihydro-9-methyl-11-[[3-[2-(1-piperidinyl)ethyl]-1-piperidinyl]carbonyl]-6H-pyrido[2,3-b][1,4]-benzodiazepin-6-on
5,11-Dihydro-11-[[3-[3-(4-morpholinyl)propyl]-1-piperidinyl]carbonyl]-6H-pyrido[2,3-b][1,4]benzodiazepin-6-on
11-[[2-[[[3-(Diethylamino)propyl]methylamino]methyl]-1-piperidinyl]carbonyl]-5,11-dihydro-6H-pyrido[2,3-b]-[1,4]benzodiazepin-6-on-dihydrochlorid
5,11-Dihydro-11-[[3-[2-(dipropylamino)ethyl]-1-piperidinyl]carbonyl]-6H-pyrido[2,3-b][1,4]benzodiazepin-6-on-hydrochlorid
5,11-Dihydro-11-[[3-[2-[(2-methylpropyl)methylamino]ethyl]-1-piperidinyl]carbonyl]-6H-pyrido[2,3-b][1,4]-benzodiazepin-6-on-hydrochlorid
5,11-Dihydro-11-[[3-[2-[(methylethyl)methylamino]ethyl]-1-piperidinyl]carbonyl]-6H-pyrido[2,3-b][1,4]-benzodiazepin-6-on
5,10-Dihydro-5-[[3-[2-(1-piperidinyl)ethyl]-1-piperidinyl]carbonyl]-11H-dibenzo[b,e][1,4]diazepin-11-on
6-Chlor-5,10-dihydro-5-[[3-[2-(1-piperidinyl)ethyl]-1-piperidinyl]carbonyl]-11H-dibenzo[b,e][1,4]diazepin-11-on
4,9-Dihydro-3-methyl-4-[[3-[2-(1-piperidinyl)ethyl]-1-piperidinyl]carbonyl]-10H-thieno[3,4-b][1,5]-benzodiazepin-10-on
1,3-Dimethyl-4-[[3-[4-(1-piperidinyl)butyl]-1-piperidinyl]carbonyl]-1,4,9,10-tetrahydropyrrolo[3,2-b][1,5]-benzodiazepin-10-on
3-Chlor-1-methyl-4-[[3-[4-(1-piperidinyl)butyl]-1-piperidinyl]carbonyl]-1,4,9,10-tetrahydropyrrolo[3,2-b][1,5]-benzodiazepin-10-on
1-Methyl-4-[[3-[4-(1-piperidinyl)butyl]-1-piperidinyl]carbonyl]-1,4,9,10-tetrahydropyrrolo[3,2-b][1,5]-benzodiazepin-10-on
4-[[3-[3-(Diethylamino)propyl]-1-piperidinyl]carbonyl]-1,3-dimethyl-1,4,9,10-tetrahydropyrazolo[4,3-b][1,5]-benzodiazepin-10-on
4-[[3-[3-(Diethylamino)propyl]-1-piperidinyl]carbonyl]-1-methyl-1,4,9,10-tetrahydropyrazolo[4,3-b][1,5]-benzodiazepin-10-on
4-[[3-[3-(Diethylamino)propyl]1-piperidinyl]carbonyl]-1-methyl-1,4,9,10-tetrahydropyrazolo[4,3-e]pyrido[3,2-b][1,4]-diazepin-10-on

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia

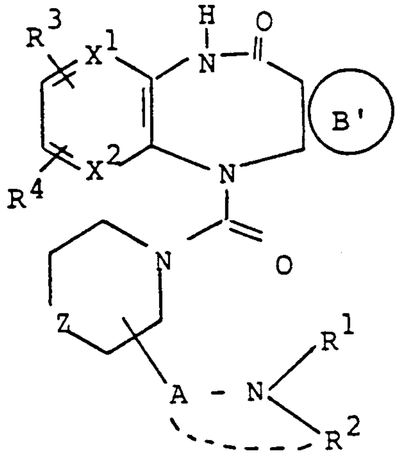

(Ia)

in der
$R^1$, $R^2$, $R^3$, $R^4$, $X^1$, $X^2$, A und Z die oben angegebenen Bedeutungen haben und

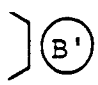

einen der zweiwertigen Reste (S), (U), (V), (W) oder (T'′)

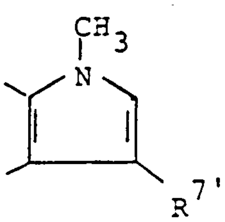

(T')

darstellt, wobei $R^{7'}$ ein Chloratom oder eine Methylgruppe ist,
erhält man durch Umsetzung von Kohlensäurederivaten der allgemeinen Formel II,

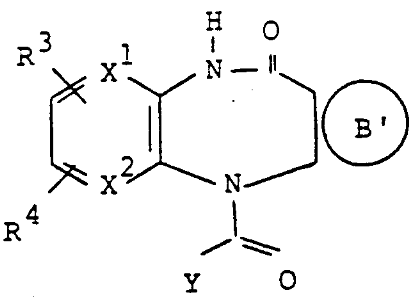

(II)

in der
$R^3$, $R^4$,

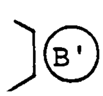

,

$X^1$, $X^2$ die angegebenen Bedeutungen haben und
Y ein Halogenatom, bevorzugt das Brom- oder Chloratom, oder den Rest $OR^{11}$ bedeutet, wobei $R^{11}$ einen

gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel III,

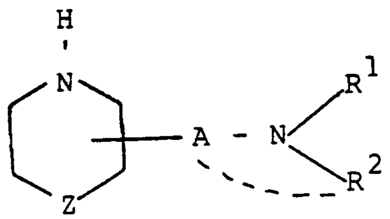
(III)

in der
$R^1$, $R^2$, A und Z wie vorstehend definiert sind.

Die Umsetzung wird ohne oder bevorzugt in Gegenwart von Lösungsmitteln, wie z. B. von Wasser, Toluol oder von Alkoholen, wie z. B Methanol, Ethanol oder Isopropanol, ganz bevorzugt jedoch in Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen -10 ° C und dem Siedepunkt des Reaktionsgemisches, bevorzugt zwischen 40 und 100 ° C durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat; von tertiären Aminen, z. B. von Triethylamin, Ethyldiisopropylamin, N,N-Dimethylanilin, Pyridin oder von 4-(Dimethylamino)pyridin; sowie die Umsetzung in Gegenwart eines Überschusses einer Verbindung der allgemeinen Formel III.

Verwendet man die Amine der allgemeinen Formel III und die Kohlensäurederivate der allgemeinen Formel II in äquimolaren Mengenverhältnissen, so erhält man - sofern Y ein Halogenatom bedeutet - direkt die halogenwasserstoffsauren Salze der gesuchten Verbindungen der allgemeinen Formel Ia.

Die Umsetzung kann jedoch auch mit einer Metallverbindung der allgemeinen Formel IIIa,

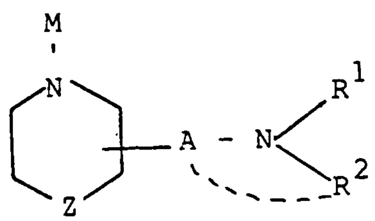
(IIIa)

in der
M ein Alkalimetallatom oder 1 Äquivalent eines Erdalkalimetallatoms bedeutet, durchgeführt werden. Dabei lassen sich Metallverbindungen der allgemeinen Formel IIIa aus III durch Umsetzung mit Alkali- oder Erdalkalimetallen, etwa mit Natrium, Kalium oder Barium, oder mit Alkali- oder Erdalkalihydriden, etwa mit Natrium-, Kalium- oder Calciumhydrid, oder durch Reaktion mit Alkali- oder Erdalkaliorganylen, z. B. mit n-Butyllithium oder Phenyllithium, in situ leicht herstellen.

b.) Basisch substituierte kondensierte Diazepinone der allgemeinen Formel Ia lassen sich auch durch Umsetzung von Tricyclen der allgemeinen Formel IV,

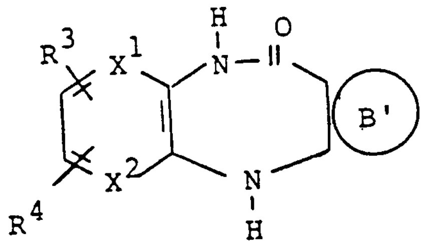

(IV)

in der
die Reste $R^3$, $R^4$, $X^1$, $X^2$ und

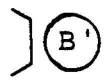

wie oben definiert sind, mit einem Chlorkohlensäurederivat der allgemeinen Formel V

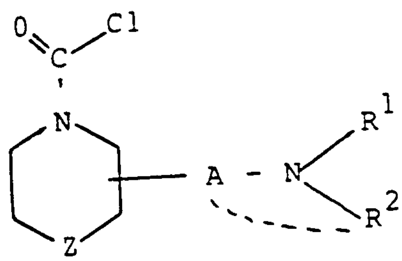

(V)

worin die Reste $R^1$, $R^2$, A und Z die oben erwähnten Bedeutungen haben, erhalten.

Die Umsetzung wird vorzugsweise in inerten organischen Lösungsmitteln, beispielsweise in aromatischen Kohlenwasserstoffen wie Toluol, Xylol, in Äthern wie Diisopropyläther, Tetrahydrofuran oder Dioxan, in Ketonen wie 3-Pentanon, in chlorierten aliphatischen Kohlenwasserstoffen, wie 1,2-Dichlorethan oder in anderen Lösungsmitteln, wie Acetonitril oder Dimethylformamid oder in Gemischen davon, gegebenenfalls in Gegenwart tertiärer organischer Basen, wie Pyridin, und bei Temperaturen bis zum Siedepunkt des Reaktionsgemisches, bevorzugt bei Temperaturen zwischen +30 und +100 °C, durchgeführt.

c) Die unter die allgemeine Formel I fallenden neuen Pyrrolo-kondensierten Diazepinone der allgemeinen Formel Ib,

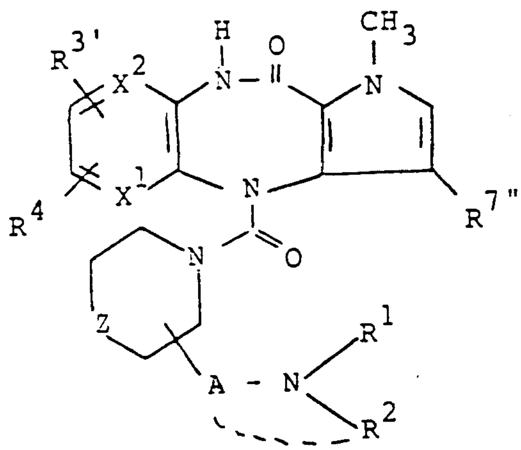

(Ib)

worin
$R^1$, $R^2$, $R^4$, $X^1$, $X^2$, A und Z die eingangs erwähnten Bedeutungen, für Z mit Ausnahme der eines

Schwefelatoms, haben,
7″ ein Wasserstoffatom und
$R^{3'}$ eine Alkylgruppe mit 1 bis 4 C-Atomen oder ein Wasserstoffatom darstellen, können auch durch Hydrogenolyse aus solchen Verbindungen der allgemeinen Formel Ib hergestellt werden, in denen $R^{7''}$ ein Chloratom bedeutet.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0 ° C bis 130 ° C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110 ° C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110 ° C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen aminoacylierten kondensierten Diazepinone der allgemeinen Formel I enthalten zum Teil ein asymmetrisches Kohlenstoffatom in der Seitenkette. Diese Verbindungen können deshalb jeweils als enantiomere (+)- und (-)Formen auftreten. Die Erfindung umfaßt die einzelnen Isomeren ebenso wie ihre Racemate.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumhydroxid oder Kaliumhydroxid neutralisiert und dadurch die entsprechende freie Verbindung in der (+)- oder (-) Form erhalten.

Jeweils nur ein Enantiomeres wird auch dadurch erhalten, daß man die oben beschriebenen Synthesen mit nur einem Enantiomeren der allgemeinen Formel III bzw. V durchführt.

Die als Zwischenprodukte erforderlichen Kohlensäurederivate der allgemeinen Formel II erhält man in Analogie zu DE-A-32 04 169, DE-A-32 04 158 und DE-A-32 04 401 durch Umsetzung von Tricyclen der allgemeinen Formel IV,

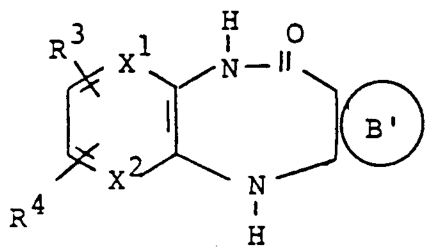

(IV)

in der

die Reste $R^3$, $R^4$, $X^1$, $X^2$ und

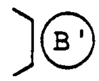

wie oben definiert sind, mit einem Halogenkohlensäurederivat der allgemeinen Formel VI,

$$\mathrm{Hal} - \underset{\underset{\displaystyle \mathrm{O}}{\|}}{\mathrm{C}} - \mathrm{Y} \qquad \mathrm{(VI)}$$

in der
Hal das Brom- oder Chloratom, bevorzugt das Chloratom, bedeutet und Y die oben angegebenen Bedeutungen hat.

Die Reaktion wird in inerten organischen Lösungsmitteln, beispielsweise aromatischen Kohlenwasserstoffen, wie Toluol, Chlorbenzol oder Xylol; offenkettigen oder cyclischen Ethern, wie Diisopropylether, Tetrahydrofuran oder Dioxan; offenkettigen oder cyclischen aliphatischen Ketonen, beispielsweise 3-Pentanon; chlorierten aliphatischen Kohlenwasserstoffen, wie 1,2-Dichlorethan oder anderen Lösungsmitteln, wie Acetonitril oder Dimethylformamid oder in Gemischen davon und bevorzugt in Gegenwart tertiärer organischer Basen, bevorzugt von Pyridin, und bei Temperaturen bis höchstens zum Siedepunkt des verwendeten Lösungsmittels oder Lösungsmittelgemisches, bevorzugt zwischen +30 und +80 °C, durchgeführt.

Zwischenverbindungen der allgemeinen Formel III, die einen in ß-Stellung zum gesättigten Heterocyclus durch ein Heteroatom unterbrochenen Alkylenrest A besitzen, können in Analogie zu im DE-A-36 26 095 ausführlich diskutierten Methoden synthetisiert werden.

Zwischenverbindungen der allgemeinen Formel III, in der Z eine Methylengruppe bedeutet, bereitet man zweckmäßig aus entsprechend substituierten Pyridinen, beispielsweise durch katalytische Hydrierung in ethanolisch-salzsaurer Lösung und unter Verwendung von Platin(IV)-oxid als Katalysator (siehe auch: F.F. Blicke u.a., J. Org. Chemistry 26, 3258 (1961)) oder in Eisessig und in Gegenwart von Platin(IV)oxid (siehe auch W.F. Minor u.a., J. Med. Pharm. Chem. 5, 96, 105 ff. (1962) und A.H. Sommers u.a., J. Amer. Chem. Soc. 75, 57, 58 ff. (1953)). Die substituierten Pyridine ihrerseits lassen sich nach dem Fachmann geläufigen Methoden leicht synthetisieren, z. B. durch Addition entsprechender sekundärer Amine, Dialkylaminoalkanole oder Dialkylaminoalkanthiole an Vinylpyridine, durch Reduktion von geeigneten Pyridinalkansäureamiden mit Lithiumaluminiumhydrid, durch Alkylierung von Picolinen mit Dialkylaminoalkylhalogeniden in Gegenwart von Lithiumdiisopropylamid oder Natriumamid (siehe auch A.E. Tschitschibabin, Bull. Soc. Chim. France 1938, 436) oder auch durch Umsetzung von (ω-Halogenalkyl)-pyridinen mit Dialkylaminoalkanolen, Dialkylaminoalkanthiolen oder sekundären Aminen (siehe auch L. Rondahl, Acta Pharm. Suec. 13, 229-34 (1976)) bzw. deren metallierten Derivaten.

Ein allgemein anwendbares Verfahren zur Synthese von Aminen der allgemeinen Formel III besteht in der Reduktion geeigneter heterocyclisch substituierter und gegebenenfalls im Alkylenrest durch Heteroatome unterbrochener Alkancarbonsäuredialkylamide, beispielsweise durch Lithiumaluminiumhydrid. Von den Vorstufen her gegebenenfalls an der Stickstoffunktion des gesättigten Heterocyclus noch vorhandene Schutzgruppen können abschließend in üblicher Weise abgespalten werden, ein Benzylrest z. B. durch Hydrogenolyse in Gegenwart von Palladium/Tierkohle. Beispielsweise kann man 5-Oxo-2-pyrrolidinessigsäure (G.L. Evans u.a., J. Amer. Chem. Soc., 72, 2727 (1950)) nacheinander mit Thionylchlorid und einem interessierenden Dialkylamin umsetzen und das so hergestellte N,N-Dialkyl-5-oxo-2-pyrrolidinacetamid anschließend mit Lithiumaluminiumhydrid zum gewünschten 2-[2-(Dialkylamino)ethyl]pyrrolidin reduzieren; oder man kann das aus 4-Benzyl-3-(hydroxymethyl)-morpholin (G.R. Brown u.a., J. Chem. Soc. Perkin Trans. I 1985, 2577) durch Einwirkung von Thionylchlorid erhältliche 4-Benzyl-3-(chlormethyl)-morpholinhydrochlorid durch Kettenverlängerung in üblicher Weise in (4-Benzyl-3-morpholinyl)alkansäuren überführen und somit zur Synthese von 3-(Dialkylaminoalkyl)morpholinen heranziehen.

Die Tricyclen der allgemeinen Formel IV sind aus der Patentliteratur bekannt oder können in enger Anlehnung an publizierte Verfahren aus gängigen Ausgangsmaterialien synthetisiert werden.
Halogenkohlensäurederivate der allgemeinen Formel VI sind bekannt.

Ein weiterer Gegenstand der Erfindung sind Arzneimittel, die ein oder mehrere basisch substituierte

Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,01 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; insbesondere besitzen sie günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vitro" Organpräparationen

Dissoziationskonstanten ($K_B$-Werte) wurden "in vitro" am Ileum und spontan schlagenden Vorhof des Meerschweinchens ermittelt. Das Ileum wurde entnommen und im Organbad in Krebs-Henseleit-Lösung inkubiert. Kontraktionen wurden derart durch steigende Konzentrationen von Methacholin (M) hervorgerufen, daß volle Konzentrations-Wirkungskurven aufgenommen werden konnten. Danach wurde M ausgewaschen, die zu untersuchende Substanz beigefügt und 30 Minuten lang in Kontakt gelassen, und wiederum eine Konzentrations-Wirkungskurve mit M aufgenommen.

Aus dem Dosisverhältnis (DR), das ist das Ausmaß der Verschiebung der Konzentrations-Wirkungskurve, wurde die Dissoziationskonstante nach Arunlakshana und Schild (Brit. J. Pharmacol. 14, 48, 1959) berechnet.

Am isolierten, spontan schlagenden rechten Vorhof reduzierte M konzentrationsabhängig die Herzfrequenz. Durch Zugabe eines Antimuscarinicums wurde dieser Effekt wieder aufgehoben. Dissoziationskonstanten für die muscarinischen Rezeptoren des Vorhofes wurden auf die gleiche Art und Weise wie oben beschrieben gewonnen. Der Vergleich der in beiden Geweben ermittelten Dissoziationskonstanten erlaubte die Identifizierung von cardioselektiven Substanzen. Die Ergebnisse sind in der Tabelle III enthalten.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. $M_1$-/$M_2$ Selektivität an der Ratte,
2. Speichselsekretionshemmende Wirkung an der Ratte und
3. Hemmung der Acetylcholinwirkung auf Blase, Bronchien und Herzfrequenz des Meerschweinchens

untersucht.

1. $M_1$-/$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle II.

2. Speichselsekretionshemmende Wirkung an der Ratte

Nach Lavy und Mulder (Arch. int. Pharmacodyn. 178, 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Am narkotisierten Meerschweinchen wurden 5 Minuten nach Gabe der Prüfsubstanz 10 µg/kg Acetylcholin intravenös als auch gleichzeitg intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle V.

B. Bindungsstudien an muscarinischen Rezeptoren:

1.) in vitro: Bestimmung des $IC_{50}$-Wertes

Als Organspender dienten männliche Sprague-Dawley-Ratten mit 180-220 g Körpergewicht. Nach Entnahme von Herz und Submandibularis wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

Gesamtherz 1: 400
Submandibularis 1: 400

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30°C. Die Inkubationsdauer betrug 45 Minuten. Als Radioligand wurde 0,3 nmolar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität die in Anwesenheit von 1 µ molar Quinclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle 1 zu ersehen.

2.) in vivo: Bestimmung der $ID_{50}$-Werte

Für diese Experimente wurden weibliche Ratten mit einem Körpergewicht von ca. 200 g verwendet. Vor Versuchsbeginn wurden die Tiere mit einer Dosis von 1,25 g/kg Urethan narkotisiert. Die Tiere erhielten jeweils die vorgesehene Dosis der Prüfsubstanz durch i.v. Injektion. Nach Ablauf von 15 Minuten wurde auf gleiche Weise 113 ng/kg $^3$H-N-Methylscopolamin ($^3$H-NMS) gegeben. Nach weiteren 15 Minuten wurden die Tiere getötet, das Herz, die Bronchien und die Tränendrüsen entnommen. Diese Organe wurden in Soluene R aufgelöst und die Radioaktivität bestimmt. Diese Messwerte wurden als Totalbindung angenommen. Der Anteil an unspezifischer Bindung wurde als jene Radioaktivität, die durch Gabe von 2 mg/kg Atropin nicht verdrängbar war definiert. Aus diesen Versuchen wurden für die einzelnen Organe $ID_{50}$-Werte ermittelt. Die $ID_{50}$-Werte sind Dosen der Prüfsubstanzen, die 50% der spezifischen Bindung von $^3$H-NMS an die muskarinischen Rezeptoren der jeweiligen Organe hemmten. Die Ergebnisse sind in der Tabelle IV enthalten.

Nach den vorstehenden Angaben wurden beispielsweise die folgenden Verbindungen untersucht:

A = 5,11-Dihydro-11-[[3-[3-(1-piperidinyl)propyl]-1-piperidinyl]carbonyl]-6H-pyrido[2,3-b][1,4]-benzodiazepin-6-on

B = 5,11-Dihydro-11-[[3-[3-(1-pyrrolidinyl)propyl]-1-piperidinyl]carbonyl]-6H-pyrido[2,3-b][1,4]-benzodiazepin-6-on-hydrochlorid

C = 5,11-Dihydro-11-[[3-[2-(1-piperidinyl)ethyl]-1-piperidinyl]carbonyl]-6H-pyrido[2,3-b][1,4]-benzodiazepin-6-on-methansulfonat

D = 5,11-Dihydro-11-[[3-[2-(1-pyrrolidinyl)ethyl]-1-piperidinyl]carbonyl]-6H-pyrido[2,3-b][1,4]-benzodiazepin-6-on-hydrochlorid

E = 11-[[3-[2-[(Cyclopentyl)methylamino]ethyl]-1-piperidinyl]carbonyl]-5,11-dihydro-6H-pyrido[2,3-b]-[1,4]-benzodiazepin-6-on-hydrochlorid

F = 5,11-Dihydro-11-[[3-[2-(hexahydro-1H-1-azepinyl)ethyl]-1-piperidinyl]carbonyl]-6H-pyrido[2,3-b][1,4]-benzodiazepin-6-on-hydrochlorid

G = 6,11-Dihydro-11-[[3-[2-(1-piperidinyl)ethyl]-1-piperidinyl]carbonyl]-5H-pyrido[2,3-b][1,5]-benzodiazepin-5-on

und als Vergleichssubstanzen

X = 11-[[2-[(Diethylamino)methyl]-1-piperidinyl]acetyl]-5,11-dihydro-6H-pyrido[2,3-b][1,4]benzodiazepin-6-on (siehe US-Patent Nr. 4 550 107)

Y = 5,11-Dihydro-11-[(4-methyl-1-piperazinyl)acetyl]-6H-pyrido[2,3-b][1,4]benzodiazepin-6-on (Pirenzepin siehe US-Patent Nr. 3 660 380)

und

Z = Atropin

Die folgenden Tabellen enthalten die dabei gefundenen Ergebnisse:

Tabelle I

| Rezeptor-Bindungs-Tests, in vitro: | | | |
|---|---|---|---|
| Substanz | Rezeptor-Bindungs-Tests -log $IC_{50}$[$nM1^{-1}$] | | Selektivitätsfaktor: Verhältnis $IC_{50}$ Submandibularis zu $IC_{50}$ Herz |
| | Herz | Submandibularis | |
| A | 8,70 | 7,70 | 10 |
| B | 8,00 | 7,40 | 4 |
| C | 8,52 | 7,52 | 10 |
| D | 8,22 | 7,30 | 8,3 |
| E | 8,52 | 7,52 | 10 |
| F | 8,52 | 7,52 | 10 |
| G | 8,40 | 7,40 | 10 |
| X | 6,82 | 5,30 | 33 |
| Y | 5,82 | 6,70 | 0,13 |
| Z | 8,40 | 8,40 | 1 |

Tabelle II

$M_1/M_2$-Selektivität und speichelsekretionshemmende Wirkung an der Ratte:

| Substanz | $M_2$-Aktivität (Ratte) $ED_{50}$[$\mu$g/kg] i.v. | $M_1$-Aktivität (Ratte) $ED_{50}$[$\mu$g/kg] i.v. | Speichelsekretionshemmung (Ratte) $ED_{50}$[$\mu$g/kg] i.v. | Verhältnis Speichelsekretionshemmung zu $M_2$-Aktivität |
|---|---|---|---|---|
| A | 6,9 | 49 | 35 | 5 |
| C | 7,6 | 39 | 58 | 7,6 |
| G | 8,13 | 7,16 | 6,83 | 20,00 |
| X | 160 | 988 | 4215 | 26,3 |
| Y | 883 | 40 | 84 | 0,1 |
| Z | 4 | 16 | 9 | 2,2 |

Tabelle III

| Dissoziationskonstanten ($K_B$-Werte) am Ileum und spontan schlagenden Vorhof des Meerschweinchens: | | |
|---|---|---|
| Substanz | $K_B$ [mol/l] | |
| | Herz | Ileum |
| A | $3{,}47 \times 10^{-10}$ | $3{,}23 \times 10^{-9}$ |
| X | $1{,}05 \times 10^{-7}$ | $6{,}17 \times 10^{-7}$ |
| Y | $2{,}4 \times 10^{-7}$ | $1{,}55 \times 10^{-7}$ |
| Z | $1{,}41 \times 10^{-9}$ | $8{,}13 \times 10^{-10}$ |

Tabelle IV

| Rezeptor-Bindungstests, in vivo: | | | | | |
|---|---|---|---|---|---|
| Substanz | $ID_{50}$ [mg/kg] | | | | Verhältnis der $ID_{50}$ Tränendrüse zu $ID_{50}$ des Atrium |
| | Herz | | Bronchien | Tränendrüsen | |
| | Atrium | Ventrikel | | | |
| A | 0,01 | 0,005 | 0,06 | 0,2 | 20 |
| X | 1,0 | 0,6 | 15,0 | > 30,0 | > 30 |
| Y | 5,0 | 1,0 | 10,0 | 10,0 | 2 |
| Z | 0,08 | 0,03 | 0,1 | 0,2 | 1,5 |

Tabelle V

| Hemmung der Acetylcholinwirkung auf Blase, Bronchien und Herzfrequenz des Meerschweinchens: Ergebnisse: | | | |
|---|---|---|---|
| Substanz | -log $ED_{50}$[$Molkg^{-1}$] | | |
| | Herz | Bronchien | Blase |
| A | 7,06 | 6,93 | 5,87 |
| B | 7,18 | 7,06 | 6,16 |
| C | 7,37 | 7,38 | 6,45 |
| D | 7,32 | 7,49 | 6,15 |
| E | 7,09 | 7,01 | 6,05 |
| F | 7,79 | 7,40 | 6,42 |
| G | 7,83 | 7,58 | 6,52 |
| X | 5,84 | 5,58 | 4,73 |
| Y | 5,85 | 6,57 | 5,36 |
| Z | 7,70 | 7,96 | 7,03 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Submandibularis.

Aus den pharmakologischen Daten der vorstehenden Tabelle II ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird. Das Verhältnis von $ED_{50}$ der Speichelsekretionshemmung zu $ED_{50}$ der $M_2$-Aktivität zeigt einen ausreichenden Sicherheitsabstand zur Nebenwirkung Mundtrockenheit für die Verbindungen A bis G. Damit ist bewiesen, daß die Substanzen A bis G bei Steigerung der Wirksamkeit (vgl. Tabelle IV) eine ausreichende, mit der Substanz X vergleichbare therapeutisch nutzbare Selektivität aufweisen.

Außerdem deuten die pharmakologischen Daten der vorstehenden Tabelle III auf ein überraschend großes Unterscheidungsvermögen zwischen Herz und glatter Muskulatur. A ist deutlich wirksamer als X und Y, wobei eine klare Selektivität zugunsten des Herzens sichtbar wird und die Verbindung A deutliche Wirksamkeitsvorteile gegenüber X und Y aufweist. Atropin (= Z) ist ein bekanntermaßen nichtselektives Antimuscarinikum und zeigt unter diesen Modellbedingungen ein umgekehrtes Selektivitätsverhältnis. Die genannten Verbindungen werden hervorragend resorbiert, da sie ein kleines Dosisverhältnis p.o. zu i.v. aufweisen. Je kleiner das Verhältnis $ED_{50}$ p.o. zu $ED_{50}$ i.v. ist, desto besser ist die Resorbierkarkeit der Wirksubstanz.

Die Tabelle IV zeigt die bevorzugte Bindung an Rezeptoren des Herzens (Atrium/Ventrikel). Die Substanz A zeigt eine entscheidende Verbesserung der Wirkungsstärke gegenüber den Substanzen X und Y am Herzen. Dies ist für die Schaffung eines vagalen Schrittmachers ein wichtiger therapeutischer Vorteil. Diese Effektivitätssteigerung gelang unter Beibehaltung des nutzbaren Selektivitätsabstandes zu den Effekten an den exokrinen Drüsen, wie sich auch aus dem Verhältnis der $ID_{50}$-Werte der Tränendrüse zu den $ID_{50}$-Werten des Atrium ergibt. Das Atropin (= Z) ist dagegen nicht selektiv.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Alle Substanzen der allgemeinen Formel I zeichnen sich durch eine ausgesprochene Hydrolyse-Stabilität aus. Dadurch wird es möglich, lagerbeständige Lösungen zur parenteralen Applikation herzustellen.

Die nachfolgenden Beispiele sollen die Erfindung näher erläutern:

"Fp." bedeutet "Schmelzpunkt", "Z." bedeutet "Zersetzung". Für alle Verbindungen liegen befriedigende Elementaranalysen, IR-, UV-, $^1$H-NMR-, häufig auch Massenspektren vor. Prozente sind, sofern nichts anderes ausdrücklich erwähnt, immer Gewichtsprozente.

## Beispiel 1

### 5,11-Dihydro-11-[[3-[3-(1-piperidinyl)propyl]-1-piperidinyl]carbonyl]-6H-pyrido[2,3-b][1,4]benzodiazepin-6-on

Zu der Suspension von 2,6 g (9,50 mMol) 11-(Chlorcarbonyl) 5,11-dihydro-6H-pyrido[2,3-b][1,4]-benzodiazepin-6-on in 30 ml Dimethylformamid tropfte man bei Zimmertemperatur und unter Rühren die Lösung von 2,00 g (9,51 mMol) 3-[3-(1-Piperidinyl)propyl]piperidin in 10 ml Dimethylformamid. Die anfangs klare Lösung trübte sich innerhalb weniger Minuten erneut ein. Nach halbstündigem Rühren bei Raumtemperatur wurde der farblose Feststoff abgenutscht und mit dreimal je 3 ml eiskaltem Ethanol gründlich ausgewaschen. Das erhaltene farblose Monohydrochlorid der gesuchten Verbindung vom Fp. > 260 °C wurde in 10 ml Wasser gelöst, mit einer gesättigten wäßrigen Kaliumcarbonatlösung bis zur deutlich alkalischen Reaktion versetzt und anschließend filtriert. Der erhaltene Feststoff wurde mit Wasser gründlich gewaschen und anschließend in Vakuumtrockenschrank bei 50 °C und über di-Phosphorpentoxid getrocknet. Man erhielt 3,0 g (71 % der Theorie) des gesuchten 5,11-Dihydro-11-[[3-[3-(1-piperidinyl)propyl]-1-piperidinyl]carbonyl]-6H-pyrido[2,3-b][1,4]benzodiazepin-6-on vom Fp. 123-125 °C, $R_f$ 0.41 (Merck DC-Fertigplatten, Kieselgel 60 $F_{254}$; Fließmittel: Dichlormethan/Methanol/wäßriger konz. Ammoniak 90/10/1, v/v/v)

Zur Überführung ins Hydrochlorid wurden 500 mg der Base in einem Gemisch aus 70 ml Essigsäurethylester und 3 ml Ethanol gelöst und mit 1,12 ml einer etherischen 1-molaren Chlorwasserstoff-Lösung versetzt. Der erhaltene Niederschlag wurde in einem Gemisch aus 50 ml Methanol und 2 ml Wasser gelöst, dann auf ein Volumen von insgesamt 10 ml im Vakuum eingedampft und mit 30 ml Aceton versetzt. Nach dem

Anreiben erhielt man farblose Kristalle, die getrocknet wurden und bei > 260 ° C schmolzen. Die Löslichkeit in Wasser betrug ca. 0,2 %.

$C_{26}H_{34}ClN_5O_2$ (484,04)

| | | | | |
|---|---|---|---|---|
| Ber.: | C 64,52 | H 7,08 | Cl 7,32 | N 14,47 |
| Gef.: | 63,99 | 7,13 | 7,21 | 14,35 |

Analog wurden 300 mg 5,11-Dihydro-11-[[3-[3-(1-piperidinyl)propyl]-1-piperidinyl]carbonyl]-6H-pyrido[2,3-b]-[1,4]benzodiazepin-6-on in 100 ml warmem Essigsäureethylester gelöst und durch Behandeln mit 60 mg Methansulfonsäure ins entsprechende Salz übergeführt. Farblose Kristalle vom Fp. 183-187 ° C, im Wasser leicht löslich.

$C_{26}H_{33}N_5O_2 \cdot CH_3SO_3H$ (543,68)

| | | | | |
|---|---|---|---|---|
| Ber.: | C 59,65 | H 6,86 | N 12,88 | S 5,90 |
| Gef.: | 59,42 | 6,75 | 12,33 | 5,91 |

Das Salz mit N-Cyclohexylsulfamidsäure schmolz bei 204-208 ° C und war im Wasser zu ca. 1 % löslich.

$C_{32}H_{46}N_6O_5S$ (611,80)

| | | | | |
|---|---|---|---|---|
| Ber.: | C 61,32 | H 7,40 | N 13,41 | S 5,11 |
| Gef.: | 60,83 | 7,44 | 13,08 | 5,16 |

## Beispiel 2

### 11-[[2-[2-(Diethylamino)ethyl]-1-piperidinyl]carbonyl]-5,11-dihydro-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-[2-(Diethylamino)ethyl]piperidin in einer Ausbeute von 37 % der Theorie. Farblose Kristalle vom Fp. 100 ° C (Z.) (aus Acetonitril), $R_f$ 0,25 (Macherey-Nagel, Polygram(R) SIL G/$UV_{254}$, pre-coated plastic sheets for TLC; Fließmittel: Dichlormethan/Essigsäureethylester/Cyclohexan/Methanol/konz. Ammoniak 57/25/8/8/1, v/v/v/v/v)

$C_{24}H_{31}N_5O_2$ (421,54)

| | | | |
|---|---|---|---|
| Ber.: | C 68,38 | H 7,41 | N 16,61 |
| Gef.: | 68,18 | 7,47 | 16,80 |

## Beispiel 3

### 5,11-Dihydro-11-[[2-[2-(dimethylamino)ethyl]-1-piperidinyl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-[2-(Dimethylamino)ethyl]piperidin in einer Ausbeute von 37 % der Theorie. Farblose Kristalle vom Fp. 188-190 ° C (aus Acetonitril), Rf 0,6 (Macherey-Nagel, Polygram(R) SIL G/$UV_{254}$, pre-coated plastic sheets for TLC; Fließmittel: Dichlormethan/Cyclohexan/Methanol/konz. Ammoniak 68/15/15/2, v/v/v/v)

$C_{22}H_{27}N_5O_2$ (393,49)

| | | | |
|---|---|---|---|
| Ber.: | C 67,15 | H 6,92 | N 17,80 |
| Gef.: | 66,97 | 6,63 | 17,83 |

## Beispiel 4

### 11-[[2-[4-(Diethylamino)butyl]-1-piperidinyl]carbonyl]-5,11-dihydro-6H-pyrido[2,3-b][1,4]benzodiazepin-6-on

Die Mischung aus 4,5 g (0,0164 mol) 11-(Chlorcarbonyl)-5,11-dihydro-6H-pyrido[2,3-b][1,4]-benzodiazepin-6-on, 2,2 g (0,02 Mol) wasserfreiem Natriumcarbonat, 4,3 g (0,0202 Mol) 2-[4-(Diethylamino)-butyl]piperidin und 100 ml Acetonitril wurde unter Rühren 1 Stunde lang unter Rückfluß gekocht. Das Lösungsmittel wurde anschließend im Vakuum abdestilliert, der verbleibende hochviskose Rückstand in 30 ml Wasser aufgenommen,natronalkalisch gestellt und mit Dichlormethan erschöpfend extrahiert. Die vereinigten Dichlormethanphasen wurden über Natriumsulfat getrocknet und eingedampft, der Rückstand an Kieselgel (35-70 mesh) unter Verwendung von Dichlormethan/Essigsäureethylester/Cyclohexan/Methanol/konz. Ammoniak 58/25/8/8/1, v/v/v/v/v, zum Eluieren chromatographisch gereinigt. Man erhielt 4,0 g (56 % der Theorie) eines farblosen Harzes, das nicht zur Kristallisation gebracht werden konnte.

$C_{26}H_{35}N_5O_2$ (449,59)

| | | | |
|---|---|---|---|
| Ber.: | C 69,46 | H 7,85 | N 15,58 |

Gef.: 69,42 7,96 15,51
Das wasserlösliche Dihydrochlorid schmolz bei 174-175 °C (Z.)
$C_{26}H_{37}Cl_2N_5O_2$ (522,51)
Ber.: C 59,77 H 7,14 Cl 13,57 N 13,40
Gef.: 60,13 7,07 13,50 13,45

Beispiel 5

5,11-Dihydro-11-[[2-[4-(dimethylamino)butyl]-1-piperidinyl]carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 2-[4-(Dimethylamino)butyl]piperidin in einer Ausbeute von 55 % der Theorie. Farblose Kristalle vom Fp. 145-147 °C (aus Essigsäureethylester), $R_f$ 0,1 (Macherey-Nagel, Polygram$^{(R)}$ SIL G/$UV_{254}$, pre-coated plastic sheets for TLC; Fließmittel: Dichlormethan/Essigsäureethylester/Cyclohexan/Methanol/konz. Ammoniak 58/25/8/8/1, v/v/v/v/v)
$C_{24}H_{31}N_5O_2$ (421,54)
Ber.: C 68,38 H 7,41 N 16,61
Gef.: 68,40 7,53 16,45

Beispiel 6

11-[[2-[3-(Diethylamino)propyl]-1-piperidinyl]carbonyl]-5,11-dihydro-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 2-[3-(Diethylamino)propyl]piperidin in einer Ausbeute von 70 % der Theorie. Farblose Kristalle vom Fp. 125-128 °C (Essigsäureethylester), $R_f$ 0,15 (Macherey-Nagel, Polygram(R) SIL G/$UV_{254}$, pre-coated plastic sheets for TLC; Fließmittel: Dichlormethan/Essigsäureethylester/Cyclohexan/Methanol/konz. Ammoniak 58/25/8/8/1, v/v/v/v/v)
$C_{25}H_{33}N_5O_2$ (435,57)
Ber.: C 68,94 H 7,64 N 16,08
Gef.: 69,00 7,66 15,90

Beispiel 7

5,11-Dihydro-11-[[2-[3-(dimethylamino)propyl]-1-piperidinyl]carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 2-[3-(Dimethylamino)propyl]piperidin in einer Ausbeute von 51 % der Theorie. Farblose Kristalle vom Fp. 142-144 °C (aus Aceton und Essigsäureethylester), $R_f$ 0.7 (Macherey-Nagel, Polygram$^{(R)}$ SIL G/$UV_{254}$, pre-coated plastic sheets for TLC; Fließmittel: Dichlormethan/Cyclohexan/Methanol/konz. Ammoniak 68/15/15/2, v/v/v/v)
$C_{23}H_{29}N_5O_2$ (407,51)
Ber.: C 67,79 H 7,17 N 17,19
Gef.: 67,80 7,27 16,99

Beispiel 8

5,11-Dihydro-11-[[4-[2-(1-piperidinyl)ethyl]-1-piperidinyl]carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 4-[2-(1-Piperidinyl)ethyl]piperidin in einer Ausbeute von 47 % der Theorie. Farblose Kristalle vom Fp. 178-180 °C.

Beispiel 9

5,11-Dihydro-11-[[4-(1-methyl-4-piperidinyl)-1-piperidinyl]carbonyl]-6H-pyrido[2,3-b][1,4]benzodiazepin-6-on-hydrochlorid

Hergestellt analog Beispiel 4 aus 11-(Chlorcarbonyl)-5,11-dihydro-6H-pyrido[2,3-b][1,4]benzodiazepin-6-on und 4-[1-Methyl-4-piperidinyl)piperidin in einer Ausbeute von 21 % der Theorie. Farblose Kristalle vom Fp. 254-255 ° C.

Beispiel 10

5,11-Dihydro-11-[[2-[2-[[1-(phenylmethyl)-4-piperidinyl]-methylamino]ethyl]-1-pyrrolidinyll-carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-[2[[1-(Phenylmethyl)-4-piperidinyl]methylaminolethyl]pyrrolidinin einer Ausbeute von 13 % der Theorie. Amorphe, harzige, farblose Substanz, $R_f$ 0,06 (Merck DC-Fertigplatten, Kieselgel 60 $F_{254}$; Fließmittel: Dichlormethan/Methanol/konz. Ammoniak 90/10/1, v/v/v)
$C_{32}H_{38}N_6O_2$ (538,69)
Ber.: C 71,35 H 7,11 N 15,60
Gef.: 70,80 7,34 15,31

Beispiel 11

5,11-Dihydro-11-[[4-[3-(1-piperidinyl)propyl]-1-piperidinyl]carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 4-[3-(1-Piperidinyl)propyl]piperidin in einer Ausbeute von 81 % der Theorie. Farblose Kristalle vom Fp. 200 ° C.

Beispiel 12

11-[[3-[3-(Diethylamino)propyl]-1-piperidinyl]carbonyl]-5,11-dihydro-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[3-(Diethylamino)propyl]piperidin in einer Ausbeute von 84 % der Theorie. Farblose Kristalle vom Fp. 257-258 ° C (Ethanol/Diisopropylether).
$C_{25}H_{34}ClN_5O_2$ (472,03)

| | | | |
|---|---|---|---|
| Ber.: C 63,61 | H 7,26 | Cl 7,51 | N 14,84 |
| Gef.: 64,00 | 7,35 | 7,44 | 14,88 |

Beispiel 13

5,11-Dihydro-11-[[3-[3-(dimethylamino)propyl]-1-piperidinyl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 3-[3-(Dimethylamino)propyl]piperidin in einer Ausbeute von 71 % der Theorie. Farblose Kristalle vom Fp. 115-117 ° C (Ethanol/Ether 1:30, v/v), $R_f$ 0,35 (Merck DC-Fertigplatten, Kieselgel 60 $F_{254}$; Fließmittel: Dichlormethan/Methanol/konz. Ammoniak 90/10/1, v/v/v)

Das Monohydrochlorid schmolz bei 222-224 ° C.
$C_{23}H_{30}ClN_5O_2$ (443,98)

| | | | |
|---|---|---|---|
| Ber.: C 62,22 | H 6,81 | Cl 7,99 | N 15,77 |
| Gef.: 62,00 | 7,07 | 7,79 | 15,58 |

Beispiel 14

5,11-Dihydro-11-[[3-[3-(4-morpholinyl)propyl]-1-piperidinyl]carbonyl]-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[3-(4-Morpholinyl)propyl]piperidin in einer Ausbeute von 61 % der Theorie. Farblose Kristalle vom

Fp. > 260 ° C (aus Ethanol/Wasser/Aceton).
$C_{25}H_{32}ClN_5O_3$ (486,01)

| | | | | |
|---|---|---|---|---|
| Ber.: | C 61,78 | H 6,64 | Cl 7,29 | N 14,41 |
| Gef.: | 61,41 | 6,68 | 7,26 | 14,18 |

Beispiel 15

5,11-Dihydro-11-[[3-[3-(hexahydro-1H-1-azepinyl)propyl]-1-piperidinyl]carbonyl]-6H-pyrido[2,3-b][1,4]-benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[3-(Hexahydro-1H-1-azepinyl)propyl]piperidin in einer Ausbeute von 60 % der Theorie. Farblose Kristalle vom Fp. > 260 ° C, $R_f$ 0,4 (Merck, DC-Fertigplatten, Kieselgel 60 $F_{254}$; Fließmittel: Dichlormethan/Methanol/konz. Ammoniak 90/10/1, v/v/v)
$C_{27}H_{36}ClN_5O_2$ (498,07)

| | | | | |
|---|---|---|---|---|
| Ber.: | C 65,11 | H 7,28 | Cl 7,12 | N 14,06 |
| Gef.: | 65,51 | 7,21 | 7,18 | 13,92 |

Beispiel 16

5,11-Dihydro-11-[[3-[3-(1-pyrrolidinyl)propyl]-1-piperidinyl]carbonyl]-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[3-(1-Pyrrolidinyl)propyl]piperidin in einer Ausbeute von 56 % der Theorie. Farblose Kristalle vom Fp. > 260 ° C (aus Wasser/Aceton), Wasserlöslichkeit ca. 0,3 %.
$C_{25}H_{32}ClN_5O_2$ (470,01)

| | | | | |
|---|---|---|---|---|
| Ber.: | C 63,89 | H 6,86 | Cl 7,54 | N 14,90 |
| Gef.: | 63,90 | 6,92 | 7,25 | 14,86 |

Beispiel 17

5,11-Dihydro-11-[[2-[4-(1-piperidinyl)butyl]-1-piperidinyl]carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 2-[4-(1-Piperidinyl)butyl]piperidin in einer Ausbeute von 41 % der Theorie. Farblose Kristalle vom Fp. 199-201 ° C, (nach zweimaligem Umkristallisieren aus Ethanol unter Verwendung von Tierkohle).
$C_{27}H_{35}N_5O_2$ (461,61)

| | | | |
|---|---|---|---|
| Ber.: | C 70,25 | H 7,64 | N 15,17 |
| Gef.: | 70,07 | 7,58 | 15,02 |

Beispiel 18

5,11-Dihydro-11-[[3-[2-(dipropylamino)ethyl]-1-piperidinyl]carbonyl]-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[2-(Dipropylamino)ethyl]piperidin in einer Ausbeute von 67 % der Theorie. Farblose Kristalle vom Fp. 202-205 ° C (Ethanol).
$C_{26}H_{36}ClN_5O_2$ (486,06)

| | | | | |
|---|---|---|---|---|
| Ber.: | C 64,25 | H 7,46 | Cl 7,29 | N 14,41 |
| Gef.: | 64,00 | 7,39 | 7,41 | 14,25 |

Beispiel 19

5,11-Dihydro-11-[[3-[4-(dimethylamino)butyl]-1-piperidinyl]carbonyl]-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[4-(Dimethylamino)butyl]piperidin in einer Ausbeute von 69 % der Theorie. Farblose Kristalle vom Fp. 234-235 ° C (Isopropanol).

$C_{24}H_{32}ClN_5O_2$ (458,0)

| | | | | |
|---|---|---|---|---|
| Ber.: | C 62,94 | H 7,04 | Cl 7,74 | N 15,29 |
| Gef.: | 63,03 | 6,90 | 7,90 | 14,89 |

Beispiel 20

5,11-Dihydro-11-[[3-[4-(1-pyrrolidinyl)butyl]-1-piperidinyl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 3-[4-(1-Pyrrolidinyl)butyl]piperidin in einer Ausbeute von 50 % der Theorie. Farblose Kristalle vom Fp. 153-156 ° C (Essigsäureethylester/Diethylether 1/1, v/v), $R_f$ 0,25 (Merck DC-Fertigplatten, Kieselgel 60 $F_{254}$; Fließmittel: Dichlormethan/Methanol/konz. Ammoniak 90/10/1, v/v/v).

$C_{26}H_{33}N_5O_2$ (447,58)

| | | | |
|---|---|---|---|
| Ber.: | C 69,77 | H 7,43 | N 15,65 |
| Gef.: | 69,72 | 7,27 | 15,25 |

Beispiel 21

6,11-Dihydro-11-[[3-[3-(1-piperidinyl)propyl]-1-piperidinyl]carbonyl]-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 3-[3-(1-Piperidinyl)propyl]piperidin in einer Ausbeute von 58 % der Theorie. Farblose Kristalle vom Fp. 165,5-167,0 ° C (Acetonitril).

$C_{26}H_{33}N_5O_2$ (447,58)

| | | | |
|---|---|---|---|
| Ber.: | C 69,77 | H 7,43 | N 15,65 |
| Gef.: | 69,65 | 7,44 | 15,50 |

Beispiel 22

11-[[3-[2-[(Cyclopentyl)methylamino]ethyl]-1-piperidinyl]carbonyl]-6,11-dihydro-5H-pyrido[2,3-b][1,5]-benzodiazepin-5-on

Hergestellt analog Beispiel 4 aus 11-(Chlorcarbonyl)-6,11-dihydro-5H-pyrido[2,3-b][1,5]benzodiazepin-5-on und 3-[2-[(Cyclopentyl)methylamino]ethyl]piperidin in einer Ausbeute von 61 % der Theorie. Farblose Kristalle vom Fp. 148-150 ° C (Acetonitril).

$C_{26}H_{33}N_5O_2$ (447,58)

| | | | |
|---|---|---|---|
| Ber.: | C 69,77 | H 7,43 | N 15,65 |
| Gef.: | 69,75 | 7,55 | 15,76 |

Beispiel 23

5,11-Dihydro-11-[[3-[2-(1-piperidinyl)ethyl]-1-piperidinyl]carbonyl]-6H-pyrido[2,3-b][1,4]benzodiazepin-6-on-methansulfonat

Hergestellt analog Beispiel 1 aus 11-(Chlorcarbonyl)-5,11-dihydro-6H-pyrido[2,3-b][1,4]benzodiazepin-6-on und 3-[2-(1-Piperidinyl)ethyl]piperidin in einer Ausbeute von 66 % der Theorie. Farblose Kristalle vom Fp. 231-234 ° C (Ethanol).

$C_{26}H_{35}N_5O_5S$ (529,65)

| | | | | |
|---|---|---|---|---|
| Ber.: | C 58,96 | H 6,66 | N 13,22 | S 6,05 |
| Gef.: | 58,84 | 6,69 | 13,07 | 6,26 |

Beispiel 24

11-[[3-[4-(Diethylamino)butyl]-1-piperidinyl]carbonyl]-5,11-dihydro-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[4-(Diethylamino)butyl]piperidin in einer Ausbeute von 90 % der Theorie. Farblose Kristalle vom Fp. 182-184 °C (Ethanol).

$C_{26}H_{36}ClN_5O_2$ (486,06)

| | | | |
|---|---|---|---|
| Ber.: C 64,25 | H 7,46 | Cl 7,29 | N 14,41 |
| Gef.: 63,80 | 7,62 | 7,35 | 13,49 |

Beispiel 25

9-Chlor-5,11-dihydro-11-[[3-[2-(1-pyrrolidinyl)ethyl]-1-piperidinyl]carbonyl]-6H-pyrido[2,3-b][1,4]-benzodiazepin-6-on

Hergestellt analog Beispiel 1 aus 9-Chlor-11-(chlorcarbonyl)5,11-dihydro-6H-pyrido[2,3-b][1,4]-benzodiazepin-6-on und 3-[2-(1-Pyrrolidinyl)ethyl]piperidin in einer Ausbeute von 42 % der Theorie. Farblose Kristalle vom Fp. 136-139 °C, $R_f$ 0,25 (Merck DC-Fertigplatten, Kieselgel 60 $F_{254}$; Fließmittel: Dichlormethan/Methanol/konz. Ammoniak 90/10/1, v/v/v).

$C_{24}H_{28}ClN_5O_2$ (453,97)

| | | | |
|---|---|---|---|
| Ber.: C 63,50 | H 6,22 | Cl 7,81 | N 15,43 |
| Gef.: 63,41 | 6,43 | 7,70 | 15,52 |

Beispiel 26

5,11-Dihydro-11-[[3-[2-(methylethylmethylamino)ethyl]-1-piperidinyl]carbonyl]-6H-pyrido[2,3-b][1,4]-benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[2-(Methylethylmethylamino)ethyl]piperidin in einer Ausbeute von 38 % der Theorie. Farblose Kristalle vom Fp. 196-198 °C (Z.), $R_f$ 0,21 (Merck DC-Fertigplatten, Kieselgel 60 $F_{254}$; Fließmittel: Dichlormethan/Methanol/konz. Ammoniak 90/10/1, v/v/v).

$C_{25}H_{34}ClN_5O_2$ (472,03)

| | | | |
|---|---|---|---|
| Ber.: C 63,61 | H 7,26 | Cl 7,51 | N 14,84 |
| Gef.: 63,55 | 7,37 | 7,61 | 14,61 |

Beispiel 27

11-[[4-[2-(Diethylamino)ethyl]-1-piperidinyl]carbonyl]-5,11-dihydro-6H-pyrido[2,3-b][1,4]benzodiazepin-6-on

Hergestellt analog Beispiel 4 aus 11-(Chlorcarbonyl)-5,11-dihydro-6H-pyrido[2,3-b]1,4]benzodiazepin-6-on und 4-[2-(Diethylamino)ethyl]piperidin in einer Ausbeute von 34 % der Theorie. Farblose Kristalle vom Fp. 154 °C (Essigsäureethylester).

Beispiel 28

5,11-Dihydro-11-[[3-[2-(1-pyrrolidinyl)ethyl]-1-piperidinyl]carbonyl]-6H-pyrido[2,3-b][1,4]benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[2-(1-Pyrrolidinyl)ethyl]piperidin in einer Ausbeute von 58 % der Theorie. Farblose Kristalle vom $R_f$ 0,25 (Merck DC-Fertigplatten, Kieselgel 60 $F_{254}$; Fließmittel: Dichlormethan/Methanol/konz. Ammoniak 90/10/1, v/v/v).

$C_{24}H_{30}ClN_5O_2$ (455,99)

| | | | |
|---|---|---|---|
| Ber.: C 63,22 | H 6,63 | Cl 7,78 | N 15,36 |
| Gef.: 63,10 | 6,76 | 7,63 | 15,21 |

Beispiel 29

11-[[3-[2-[(Cyclopentyl)methylamino]ethyl]-1-piperidinyl]carbonyl]-5,11-dihydro-6H-pyrido[2,3-b][1,4]-benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[2-[(Cyclopentyl)methylamino]ethyl]piperidin in einer Ausbeute von 54 % der Theorie. Farblose Kristalle vom Fp. 244-246 °C (Ethanol), $R_f$ 0,31 (Merck DC-Fertigplatten, Kieselgel 60 $F_{254}$; Fließmittel: Dichlormethan/Methanol/konz. Ammoniak 90/10/1, v/v/v).

$C_{26}H_{34}ClN_5O_2$ (484,04)

| | | | | |
|---|---|---|---|---|
| Ber.: | C 64,52 | H 7,08 | Cl 7,32 | N 14,47 |
| Gef.: | 64,33 | 7,11 | 7,53 | 14,39 |

Beispiel 30

5,11-Dihydro-11-[[3-[2-(hexahydro-1H-1-azepinyl)ethyl]-1-piperidinyl]carbonyl]-6H-pyrido[2,3-b][1,4]-benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[2-(Hexahydro-1H-1-azepinyl)ethyl]piperidin in einer Ausbeute von 78 % der Theorie. Farblose Kristalle vom Fp. 255-257 °C (Ethanol/Wasser/Aceton 45/5/50, v/v/v), $R_f$ 0,27 (Merck DC-Fertigplatten, Kieselgel 60 $F_{254}$; Fließmittel: Dichlormethan/Methanol/konz. Ammoniak 90/10/1, v/v/v).

$C_{26}H_{34}ClN_5O_2$ (484,04)

| | | | | |
|---|---|---|---|---|
| Ber.: | C 64,52 | H 7,08 | Cl 7,32 | N 14,47 |
| Gef.: | 64,39 | 7,17 | 7,50 | 14,29 |

Beispiel 31

5,11-Dihydro-11-[[3-[2-(methylethylethylamino)ethyl]-1-piperidinyl]carbonyl]-6H-pyrido[2,3-b][1,4]-benzodiazepin-6-on-hydrochlorid

Hergestellt analog Beispiel 1 aus 11-(Chlorcarbonyl)-5,11-dihydro-6H-pyrido[2,3-b][1,4]benzodiazepin-6-on und 3-[2-Methylethylethylamino)ethyl]piperidin in einer Ausbeute von 43 % der Theorie. Farblose Kristalle vom Fp. 228-230 °C, $R_f$ 0,39 (Merck DC-Fertigplatten, Kieselgel 60 $F_{254}$; Fließmittel: Dichlormethan/Methanol/konz. Ammoniak 90/10/1, v/v/v).

$C_{25}H_{34}ClN_5O_2$ (472,03)

| | | | | |
|---|---|---|---|---|
| Ber.: | C 63,61 | H 7,26 | Cl 7,51 | N 14,84 |
| Gef.: | 63,27 | 7,40 | 7,81 | 14,80 |

Beispiel 32

6,11-Dihydro-11-[[3-[3-(1-pyrrolidinyl)propyl]-1-piperidinyl]carbonyl]-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 3-[3-(1-Pyrrolidinyl)propyl]piperidin in einer Ausbeute von 56 % der Theorie. Farblose Kristalle vom Fp. 174-176 °C (Acetonitril), $R_f$ 0,65 (Macherey-Nagel, Polygram(R) SIL G/$UV_{254}$, pre-coated plastic sheets for TLC; Fließmittel: Dichlormethan/Cyclohexan/Methanol/konz. Ammoniak 68/15/15/2, v/v/v/v)

$C_{25}H_{31}N_5O_2$ (433,55)

| | | | |
|---|---|---|---|
| Ber.: | C 69,26 | H 7,21 | N 16,15 |
| Gef.: | 69,20 | 7,19 | 16,45 |

Beispiel 33

6,11-Dihydro-11-[[3-[2-(hexahydro-1H-1-azepinyl)ethyl]-1-piperidinyl]carbonyl]-5H-pyrido[2,3-b][1,5]-benzodiazepin-5-on

Hergestellt analog Beispiel 4 aus 11-(Chlorcarbonyl)-6,11-dihydro-5H-pyrido[2,3-b][1,5]benzodiazepin-5-on und 3-[2-(Hexahydro-1H-1-azepinyl)ethyl]piperidin in einer Ausbeute von 58 % der Theorie. Farblose Kristalle vom Fp. 152-154 °C (Acetonitril), $R_f$ 0,64 (Macherey-Nagel, Polygram(R) SIL G/$UV_{254}$, pre-coated plastic sheets for TLC; Fließmittel: Dichlormethen/Cyclohexan/Methanol/konz. Ammoniak 68/15/15/2, v/v/v/v)

$C_{26}H_{33}N_5O_2$ (447,58)

| | | | |
|---|---|---|---|
| Ber.: | C 69,77 | H 7,43 | N 15,65 |
| Gef.: | 69,74 | 7,51 | 15,64 |

Beispiel 34

6,11-Dihydro-11-[[4-[4-(1-pyrrolidinyl)butyl]-1-piperidinyl]-carbonyl]-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 4-[4]-(1-Pyrrolidinyl)butyl]piperidin in einer Ausbeute von 72 % der Theorie. Farblose Kristalle vom Fp. 193-195 ° C (Ethanol).

$C_{26}H_{33}N_5O_2$ (447,58)

| | | | |
|---|---|---|---|
| Ber.: | C 69,77 | H 7,43 | N 15,65 |
| Gef.: | 69,97 | 7,45 | 15,78 |

Beispiel 35

4-[[3-[3-(Diethylamino)propyl]-1-piperidinyl]carbonyl]-4,9-dihydro-3-methyl-10H-thieno[3,4-b][1,5]-benzodiazepin-10-on

Hergestellt analog Beispiel 4 aus 4-(Chlorcarbonyl)-4,9-dihydro-3-methyl-10H-thieno[3,4-b]1,5]-benzodiazepin-l0-on und 3-[3-(Diethylamino)propyl]piperidin in einer Ausbeute von 34 % der Theorie. Farblose Kristalle vom Fp. 136,0-137,5 ° C (Diisopropylether).

Beispiel 36

4,9-Dihydro-4-[[3-[3-(dimethylamino)propyl]-1-piperidinyl]carbonyl]-3-methyl-10H-thieno[3,4-b][1,5]-benzodiazepin-10-on

Hergestellt analog Beispiel 4 aus 4-(Chlorcarbonyl)-4,9-dihydro-3-methyl-10H-thieno[3,4-b][1,5]-benzodiazepin-l0-on und 3-[3-(Dimethylamino)propyl]piperidin in einer Ausbeute von 35 % der Theorie. Farblose Kristalle vom Fp. 137-139 ° C (Acetonitril).

$C_{23}H_{30}N_4O_2S$ (426,58)

| | | | | |
|---|---|---|---|---|
| Ber.: | C 64,76 | H 7,09 | N 13,13 | S 7,52 |
| Gef.: | 64,77 | 7,25 | 13,07 | 7,75 |

Beispiel 37

6,11-Dihydro-11-[[3-[3-(1-piperidinyl)propyl]-1-piperidinyl]-carbonyl]-5H-pyrido[2,3-b][1,5]benzodiazepin-5-on

In eine Mischung, bestehend aus 22,5 ml einer 20 %igen Lösung von Phosgen in Toluol, 100 ml Acetonitril und 4,75 g (0,045 Mol) wasserfreiem Natriumcarbonat wurden unter äußerer Kühlung mit Eis 8,94 g (0,0425 mol) 3-[3-(1-Piperidinyl)propyl]piperidin zugetropft. Man rührte noch 60 Minuten bei Zimmertemperatur, trug dann 9,0 g (0,0428 Mol) 6,11-Dihydro-5H-pyrido[2,3-b]1,5]benzodiazepin-5-on in die Reaktionsmischung ein und kochte anschließend 4 Stunden unter Rückfluß. Man filtrierte die siedend heiße Mischung, wusch den Niederschlag mit dreimal je 10 ml heißem Acetonitril gründlich durch und engte die vereinigten Filtrate im Vakuum auf ein Gesamtvolumen von 50 ml ein. Man ließ erkalten und hielt unter gelegentlichem Umrühren mit einem Glasstab 2 Stunden bei 0 bis 5 ° C, nutschte den entstandenen Kristallbrei ab, kristallisierte nochmals aus Acetonitril um und erhielt farblose Kristalle vom Fp. 166-167 ° C, nach Mischschmelzpunkt, IR- und $^1$H-NMR-Spektrum identisch mit einem nach Beispiel 21 hergestellten Präparat. Ausbeute: 6,2 g (33 % der Theorie).

Entsprechend erhielt man:

5,11-Dihydro-11-[[3-[3-(1-piperidinyl)propyl]-1-piperidinyl]-carbonyl]-6H-pyrido[2,3-b][1,4]benzodiazepin-6-on vom Fp. 123,5-125,5 ° C (Acetonitril);

11-[[3-[3-(Diethylamino)propyl]-1-piperidinyl]carbonyl]-5,11-dihydro-6H-pyrido[2,3-b][1,4]benzodiazepin-6-on, das nach der Überführung ins Monohydrochlorid bei 257,5-259,0 ° C (Ethanol) schmolz;

11-[[3-[2-[(Cyclopentyl)methylamino]ethyl]-1-piperidinyl]-carbonyl]-6,11-dihydro-5H-pyrido[2,3-b][1,5]-benzodiazepin-5-on vom Fp. 149-150 ° C (Acetonitril);

5,11-Dihydro-11-[[3-[2-(1-piperidinyl)ethyl]-1-piperidinyl]-carbonyl]-6H-pyrido[2,3-b][1,4]benzodiazepin-6-on, das nach der Überführung ins Methansulfonat bei 233-234 ° C (Ethanol) schmolz.

Beispiel 38

5,11-Dihydro-11-[[3-2-(1-piperidinyl)ethyl]-1-pyrrolidinyl]carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 3-[2-(1-Piperidinyl)ethyl]pyrrolidin in einer Ausbeute von 75 % der Theorie. Farblose Kristalle vom Fp. 234-236 ° C (Methanol).
$C_{24}H_{29}N_5O_2$ (419,53)
Ber.: C 68,71 H 6,97 N 16,69
Gef.: 68,38 7,05 16,70

Beispiel 39

6,11-Dihydro-11-[[3-[3-(4-morpholinyl)propyl]-1-piperidinyl]carbonyl]-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 3-[3-(4-Morpholinyl)propyl]piperidin in einer Ausbeute von 43 % der Theorie. Farblose Kristalle vom Fp. 161-163 ° C (Acetonitril).
$C_{25}H_{31}N_5O_3$ (449,55)
Ber.: C 66,79 H 6,95 N 15,58
Gef.: 66,21 6,99 15,63
Das Monohydrochlorrid schmolz bei 221-223 ° C.

Beispiel 40

6,11-Dihydro-11-[[3-[2-(1-piperidinyl)ethyl]-1-piperidinyl] carbonyl]-5H-pyrido[2,3-b][1,5]benzodiazepin-5-on

Hergestellt analog Beispiel 4 aus 11-(Chlorcarbonyl) 6,11-dihydro-5H-pyrido[2,3-b][1,5]benzodiazepin-5-on und 3-[2-(1-Piperidinyl)ethyl]piperidin in einer Ausbeute von 50 % der Theorie. Farblose Kristalle vom Fp. 168-169 ° C (Acetonitril).
$C_{25}H_{31}N_5O_2$ (433,55)
Ber.: C 69,26 H 7,21 N 16,15
Gef.: 69,22 7,20 16,04

Beispiel 41

11-[[3-[2-(Diethylamino)ethyl]-1-piperidinyl]carbonyl]-6,11-dihydro-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 3-[2-(Diethylaminol)ethyl]piperidin in einer Ausbeute von 54 % der Theorie. Farblose Kristalle vom Fp. 110-113 ° C (Acetonitril).
$C_{24}H_{31}N_5O_2$ (421,54)
Ber.: C 68,38 H 7,41 N 16,61
Gef.: 69,00 7,73 16,56

Beispiel 42

11-[[3-[3-(Diethylamino)propyl]-1-piperidinyl]carbonyl]-6,11-dihydro-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 3-[3-(Diethylaminol)propyl]piperidin in einer Ausbeute von 38 % der Theorie. Farblose Kristalle vom Fp. 123-125 ° C (Acetonitril).
$C_{25}H_{33}N_5O_2$ (435,57)
Ber.: C 68,94 H 7,64 N 16,08
Gef.: 68,80 7,64 15,79

Beispiel 43

11-[[4-[4-(Diethylamino)butyl]-1-piperidinyl]carbonyl]-5,11-dihydro-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 4-[4-(Diethylamino)butyl]piperidin in einer Ausbeute von 62 % der Theorie. Farblose Kristalle vom Fp. 126-127°C.
$C_{26}H_{35}N_5O_2$ (449,59)
Ber.: C 69,46 H 7,85 N 15,58
Gef.: 69,18 7,86 15,70

Beispiel 44

9-Chlor-11-[[4-[4-(diethylamino)butyl]-1-piperidinyl]carbonyl]-5,11-dihydro-6H-pyrido[2,3-b][1,4]-benzodiazepin-6-on

Hergestellt analog Beispiel 4 aus 9-Chlor-11-(chlorcarbonyl)5,11-dihydro-6H-pyrido[2,3-b][1,4]-benzodiazepin-6-on und 4-[4-(Diethylamino)butyl]piperidin in einer Ausbeute von 38 % der Theorie. Farblose Kristalle vom Fp. 126-127°C.
$C_{26}H_{34}ClN_5O_2$ (484,04)
Ber.: C 64,52 H 7,08 N 14,47 Cl 7,32
Gef.: 64,12 7,23 14,53 7,37

Beispiel 45

3-Methyl-4-[[4-[4-(1-piperidinyl)butyl]-1-piperidinyl]carbonyl]-4,9-dihydro-10H-thieno[3,4-b][1,5]-benzodiazepin-10-on

Hergestellt analog Beispiel 4 aus 4-(Chlorcarbonyl)-4,9-dihydro-3-methyl-10H-thieno[3,4-b][1,5]-benzodiazepin-10-on und 4-[4-(1-Piperidinyl)butyl]piperidin in einer Ausbeute von 31 % der Theorie. Farblose Kristalle vom Fp. 209-210°C (Essigsäureethylester).

Beispiel 46

11-[[2-[2-[[3-(Diethylamino)propyl]methylamino]ethyl]-1-piperidinyl]carbonyl]-5,11-dihydro-6H-pyrido[2,3-b]-[1,4]benzodiazepin-6-on

Hergestellt analog Beispiel 4 aus 11-(Chlorcarbonyl)-5,11-dihydro-6H-pyrido[2,3-b][1,4]benzodiazepin-10-on und 2-[2-[[3-(Diethylamino)propyl]methylamino]ethyl]piperidin (Sdp.$_{0.1\ mmHg}$ 130-134°C) in einer Ausbeute von 80 % der Theorie. Farblose Kristalle vom Fp. 123-125°C (Acetonitril).
$C_{28}H_{40}N_6O_2$ (492,66)
Ber.: C 68,26 H 8,18 N 17,06
Gef.: 68,08 8,30 17,34

Beispiel 47

11-[[2-[2-[2-(Diethylamino)ethoxy]ethyl]-1-piperidinyl]carbonyl]-5,11-dihydro-6H-pyrido[2,3-b][1,4]-benzodiazepin-6-on

Hergestellt analog Beispiel 4 aus 11-(Chlorcarbonyl)-5,11-dihydro-6H-pyrido[2,3-b][1,4]benzodiazepin-6-on und 2-[2-[2-(Diethylamino)ethoxy]ethyl]piperidin (Sdp.$_{0,4\ mmHg}$ 95-99°C) in einer Ausbeute von 79 % der Theorie. Farblose Kristalle vom Fp. 134-136°C (Acetonitril).
$C_{26}H_{35}N_5O_3$ (465,59)
Ber.: C 67,07 H 7,58 N 15,04
Gef.: 67,14 7,64 15,16

Beispiel 48

4-[[2-[2-[[2-(Diethylamino)ethyl]methylamino]ethyl]-1-piperidinyl]carbonyl]-4,9-dihydro-3-methyl-10H-thieno-[3,4-b][1,5]benzodiazepin-10-on

Hergestellt analog Beispiel 4, jedoch unter Verwendung von Dichlormethan an Stelle von Acetonitril als

Lösungsmittel, aus 4-(Chlorcarbonyl) 4,9-dihydro-3-methyl-10H-thieno-[3,4-b][1,5]benzodiazpin-10-on und 2-[2-[[2-(Diethylamino)-ethyl]methylamino]ethyl]piperidin (Sdp.$_{0,3\ mmHg}$ 92-95 ° C) in einer Ausbeute von 22 % der Theorie. Farblose Kristalle vom Fp. 131-133 ° C (Acetonitril).
$C_{27}H_{39}N_5O_2S$ (497,70)

| | | | |
|---|---|---|---|
| Ber.: C 65,16 | H 7,90 | N 14,07 | S 6,44 |
| Gef.: 65,00 | 7,70 | 14,00 | 6,63 |

Beispiel 49

4-[[2-[2-[2-(Diethylamino)ethoxy]ethyl]-1-piperidinyl]-carbonyl]-4,9-dihydro-3-methyl-10H-thieno[3,4-b][1,5]-benzodiazepin-10-on

Hergestellt analog Beispiel 4 aus 4-(Chlorcarbonyl)-3-methyl-10H-thieno[3,4-b][1,5]benzodiazepin-10-on und 2-[2-[2-(Diethylamino)ethoxy]ethyl]piperidin in einer Ausbeute von 53 % der Theorie. Farblose Kristalle vom Fp. 105-107 ° C (Diisopropylether).
$C_{26}H_{36}N_4O_3S$ (484,66)

| | | | |
|---|---|---|---|
| Ber.: C 64,43 | H 7,49 | N 11,56 | S 6,62 |
| Gef.: 64,00 | 7,44 | 11,70 | 6,60 |

Beispiel 50

11-[[2-[2-[[3-(Diethylamino)propyl]methylamino]ethyl]-1-piperidinyl]carbonyl]-6,11-dihydro-5H-pyrido[2,3-b]-[1,5]-benzodiazepin-5-on-dihydrochlorid-hydrat

Hergestellt analog Beispiel 4 aus 11-(Chlorcarbonyl)-6,11-dihydro-5H-pyrido[2,3-b][1,5]benzodiazepin-5-on und 2-[2-[[3-(Diethylamino)propyl]methylamino]ethyl]piperidin in einer Ausbeute von 48 % der Theorie. Das farblose Dihydrochlorid-hydrat schmolz bei 145-150 ° C (Z.).
$C_{28}H_{40}N_6O_2 x 2HCl x H_2O$ (583,60)

| | | | |
|---|---|---|---|
| Ber.: C 57,63 | H 7,60 | Cl 12,15 | N 14,40 |
| Gef.: 57,64 | 7,80 | 12,05 | 14,49 |

Beispiel 51

11-[[3-[2-(Diethylamino)ethyl]-1-pyrrolidinyl]carbonyl]-5,11-dihydro-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 3-[2-[Diethylamino)ethyl]pyrrolidin in einer Ausbeute von 13 % der Theorie. Farblose Kristalle vom Fp. 137-138 ° C (aus Essigsäureethylester/Methanol 98/2 v/v.).
$C_{23}H_{29}N_5O_2$ (407,51)

| | | |
|---|---|---|
| Ber.: C 67,79 | H 7,17 | N 17,19 |
| Gef.: 67,19 | 7,05 | 17,15 |

Beispiel 52

5,11-Dihydro-11-[[4-[3-(4-methyl-1-piperazinyl)propyl]-1-piperidinyl]carbonyl]-6H-pyrido[2,3-b][1,4]-benzodiazepin-6-on

Hergestellt analog Beispiel 4 aus 11-(Chlorcarbonyl)-5,11-dihydro-6H-pyrido[2,3-b][1,4]benzodiazepin-6-on und 4-[3-(4-methyl-1-piperazinyl)propyl]piperidin in einer Ausbeute von 17 % der Theorie. Farblose Kristalle vom Fp. 213-214 ° C; $R_f$ 0.25 (Merck, DC-Fertigplatten, Kieselgel 60 $F_{254}$; Fließmittel: Essigsäureethylester/Methanol/Cyclohexan/konz. Ammoniak 80/10/10/1 v/v/v/v).

Beispiel 53

4-[[4-[2-[2-(Diethylamino)ethoxy]ethyl]-1-piperidinyl]carbonyl]-4,9-dihydro-3-methyl-10H-thieno[3,4-b][1,5]-benzodiazepin-10-on

Hergestellt analog Beispiel 4 aus 4-(Chlorcarbonyl)-4,9-dihydro-3-methyl-10H-thieno[3,4-b][1,5]-

benzodiazepin-10-on und 4-[2-[2-(Diethylamino)ethoxy]ethyl]piperidin (Sdp.$_{0,009\ mmHg}$ 101-102°C) in einer Ausbeute von 71 % der Theorie. Farblose Kristalle vom Fp.125-126°C (Diisopropylether).
$C_{26}H_{36}N_4O_3S$ (484,66)

| | | | | |
|---|---|---|---|---|
| Ber.: | C 64,43 | H 7,49 | N 11,56 | S 6,62 |
| Gef.: | 64,50 | 7,27 | 11,80 | 6,52 |

Beispiel 54

11-[[4-[2-[2-(Diethylamino)ethoxy]ethyl]-1-piperidinyl]carbonyl]-5,11-dihydro-6H-pyrido[2,3-b][1,4]-benzodiazepin-6-on

Hergestellt analog Beispiel 4 aus 11-(Chlorcarbonyl)-5,11-dihydro-6H-pyrido[2,3-b][1,4]benzodiazepin-6-on und 4-[2-[2-(Diethylamino)ethoxy]ethyl]piperidin in einer Ausbeute von 71 % der Theorie. Farblose Kristalle vom Fp. 119-120°C (Cyclohexan), $R_f$ 0,43 (Macherey-Nagel, Polygram$^{(R)}$ SIL G/UV$_{254}$, pre-coated plastic sheets for TLC; Fließmittel: Essigsäureethylester/Methanol/konz. Ammoniak 100/30/3 v/v/v)

Beispiel 55

9-Chlor-11-[[4-[2-[2-(diethylamino)ethoxy]ethyl]-1-piperidinyl]carbonyl]-5,11-dihydro-6H-pyrido[2,3-b][1,4]-benzodiazepin-6-on

Hergestellt analog Beispiel 4 aus 9-Chlor-11-(chlorcarbonyl)5,11-dihydro-6H-pyrido[2,3-b][1,4]-benzodiazepin-6-on und 4-[2-[2-(Diethylamino)ethoxy]ethyl]piperidin in einer Ausbeute von 43 % der Theorie. Farblose Kristalle vom Fp. 145-146°C (Acetonitril), $R_f$ 0,39 (Bedingungen wie in Beispiel 54).
$C_{26}H_{34}ClN_5O_3$ (500,04)

| | | | | |
|---|---|---|---|---|
| Ber.: | C 62,45 | H 6,85 | Cl 7,09 | N 14,01 |
| Gef.: | 62,16 | 6,97 | 7,30 | 14,12 |

Beispiel 56

11-[[4-[2-[2-Diethylamino)ethoxy]ethyl]-1-piperidinyl]carbonyl]-6,11-dihydro-5H-pyrido[2,3-b][1,5]-benzodiazepin-5-on

Hergestellt analog Beispiel 4 aus 11-(Chlorcarbonyl)-6,11-dihydro-5H-pyrido[2,3-b][l,5]benzodiazepin-5-on und 4-[2-[2-(Diethylamino)ethoxy]ethyl]piperidin in einer Ausbeute von 83 % der Theorie. Das farblose Hydrochlorid schmolz bei 148-150°C (Essigsäureethylester).
$C_{26}H_{36}ClN_5O_3$ (502,05)

| | | | | |
|---|---|---|---|---|
| Ber.: | C 62,20 | H 7,23 | Cl 7,06 | N 13,95 |
| Gef.: | 61,96 | 7,37 | 7,20 | 13,77 |

Beispiel 57

5,11-Dihydro-11-[[2-[2-(1-piperidinyl)ethyl] 4-morpholinyl] carbonyl]-6H-pyrido[2,3-b][1,4]benzodiazepin-6-on

Hergestellt analog Beispiel 4 aus 11-(Chlorcarbonyl)-5,11-dihydro-6H-pyrido[2,3-b][1,4]benzodiazepin-6-on und 2-[2-(1-Piperidinyl)ethyl]morpholin ($R_f$ 0,4 [Macherey-Nagel, Polygram$^{(R)}$ SIL G/UV$_{254}$, pre-coated plastic sheets for TLC; Fließmittel: Dichlormethan/Methanol/Cyclohexan/konz. Ammoniak 68/15/15/2, v/v/v/v]) in einer Ausbeute von 79 % der Therorie. Das farblose Hydrochlorid schmolz bei 274-275°C.
$C_{24}H_{30}ClN_5O_3$ (471,99)

| | | | | |
|---|---|---|---|---|
| Ber.: | C 61,07 | H 6,41 | Cl 7,51 | N 14,81 |
| Gef.: | 60,86 | 6.37 | 7,69 | 15,08 |

Beispiel 58

6,11-Dihydro-11-[[2-[2-(1-piperidinyl)ethyl]-4-morpholinyl]carbonyl]-5H-pyrido[2,3-b][1,5]benzodiazepin-5-on

Hergestellt analog Beispiel 4 aus 11-(Chlorcarbonyl)-6,11-dihydro-5H-pyrido[2,3-b][1,5]benzodiazepin-5-on und 2-[2-(1-Piperidinyl)ethyl]morpholin in einer Ausbeute von 78 % der Theorie. Farblose Kristalle vom

Fp. 136-138 °C (Diisopropylether), $R_f$ 0,46 (Macherey-Nagel, Polygram(R) SIL G/$UV_{254}$, pre-coated plastic sheets for TLC; Fließmittel: Essigsäureethylester/Methanol/konz. Ammoniak 100/30/3. v/v/v).
$C_{24}H_{29}N_5O_3$ (435,52)
Ber.: C 66,19 H 6,71 N 16,08
Gef.: 66,01 6.64 16,10

Beispiel 59

5,11-Dihydro-8-methyl-11-[[2-[2-(1-piperidinyl)ethyl]-4-morpholinyl]carbonyl]-6H-pyrido[2,3-b][1,4]-benzodiazepin-6-on

Hergestellt analog Beispiel 4 aus 11-(Chlorcarbonyl)-5,11-dihydro-8-methyl-6H-pyrido[2,3-b][1,4]-benzodiazepin-6-on und 2-[2-(1-Piperidinyl)ethyl]morpholin in einer Ausbeute von 79 % der Theorie. $R_f$ 0,43 (Bedingungen wie in Beispiel 58). Das farblose Hydrochlorid schmolz bei 272-273 °C unter Zersetzung.
$C_{25}H_{32}ClN_5O_3$ (486,01)
Ber.: C 61,78 H 6,64 Cl 7,29 N 14,41
Gef.: 61,49 6,65 7,43 14,50

Beispiel 60

11-[[2-[2-[2-(Diethylamino)ethoxy]ethyl]-1-piperidinyl]carbonyl]-6,11-dihydro-5H-pyrido[2,3-b][1,5]-benzodiazepin-5-on

Hergestellt analog Beispiel 4 aus 11-(Chlorcarbonyl)-6,11-dihydro-5H-pyrido[2,3-b][1,5]benzodiazepin-5-on und 2-[2-[2-(Diethylamino)ethoxy]ethyl]piperidin in einer Ausbeute von 86 % der Theorie. Das farblose Hydrochlorid schmolz bei 186-187 °C.
$C_{26}H_{36}ClN_5O_3$ (502,05)
Ber.: C 62,20 H 7,23 Cl 7,06 N 13,95
Gef.: 61,91 7,13 7,09 13,90

Beispiel 61

4,9-Dihydro-3-methyl-4-[[2-[2-(1-piperidinyl)ethyl]-4-morpholinyl]carbonyl]-10H-thieno[3,4-b][1,5]-benzodiazepin-10-on

Hergestellt analog Beispiel 4 aus 4-(Chlorcarbonyl)-4,9-dihydro-3-methyl 10H-thieno[3,4-b][1,5]-benzodiazepin-10-on und 2-[2-(1-Piperidinyl)ethyl]morpholin in einer Ausbeute von 74 % der Theorie. $R_f$ 0,48 (Bedingungen wie in Beispiel 58). Das farblose Hydrochlorid schmolz bei 254-256 °C (Z.).
$C_{24}H_{31}ClN_4O_3S$ (491,05)
Ber.: C 58,70 H 6,36 Cl 7,22 N 11,41 S 6,53
Gef.: 58,51 6,38 7,31 11,56 6,71
Die folgenden Beispiele veranschaulichen die Herstellung pharmazeutischer Zubereitungsformen:

Beispiel I

Tabletten mit 5 mg 5,11-Dihydro-11-[[3-[3-(1-piperidinyl)propyl]-1-piperidinyl]carbonyl]-6H-pyrido[2,3-b][1,4]-benzodiazepin-6-on

Zusammensetzung: 1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 ° C getrocknet, nochmals durch obiges Sieb gerieben, mit Magnesiumstearat vermischt und zu Tabletten verpreßt.
Tablettengewicht: 220 mg
Stempel: 9 mm

Beispiel II

Dragées mit 5 mg 5,11-Dihydro-11-[[3-[3-(1-piperidinyl)propyl]-1-piperidinyl]carbonyl]-6H-pyrido[2,3-b][1,4]-benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 5,11-Dihydro-11-[[3-[3-(1-piperidinyl)propyl]-1-piperidinyl]carbonyl]-6H-pyrido[2,3-b]-[1,4]benzodiazepin-6-on-methansulfonat

Zusammensetzung: 1 Ampulle enthält:
Wirkstoff 10,0 mg
Natriumchlorid 8,0 mg
Dest. Wasser ad 1 ml

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120 ° C.

Beispiel IV

Suppositorien mit 20 mg 5,11-Dihydro-11-[[3-[3-(1-piperidinyl)propyl]-1-piperidinyl]carbonyl]-6H-pyrido[2,3-b][1,4]benzodiazepin-6-on

Zusammensetzung:

| | |
|---|---|
| 1 Zäpfchen enthält: | |
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40 ° C abgekühlten Zäpfchenmasse suspendiert. Man gießt die Masse bei 37 ° C in leicht vorgekühlte Zäpfchenformen aus.
Zäpfchengewicht 1,7 g

Beispiele V

Tropfen mit 5,11-Dihydro-11-[[3-[3-(1-piperidinyl)propyl]-1-piperidinyl]carbonyl]-6H-pyrido[2,3-b][1,4]-benzodiazepin-6-on-methansulfonat

Zusammensetzung:

100 ml Tropflösung entnalten:

| | |
|---|---|
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Patentansprüche**

**Patentansprüche für folgende Vertragsstaaten : AT, BE, CH, DE, FR, GB, IT, LI, LU, NL, SE**

**1.** Kondensierte Diazepinone der allgemeinen Formel I

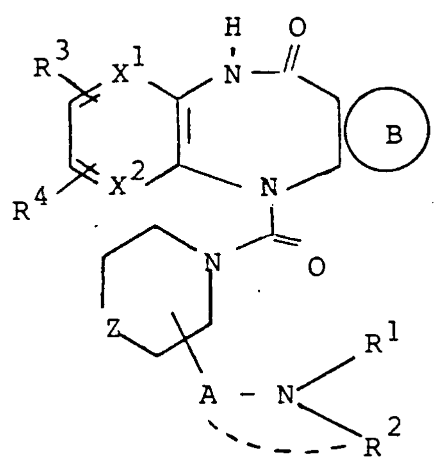

(I)

in der

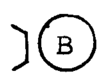

einen der zweiwertigen Reste

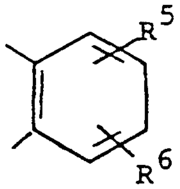 , 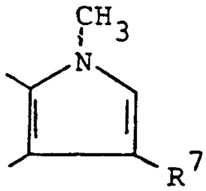 , 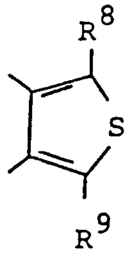 , 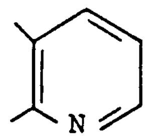 oder 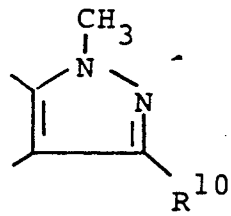

(S) (T) (U) (V) (W)

darstellt und
$X^1$, $X^2$, A, $R^1$ bis $R^{10}$ und Z die folgenden Bedeutungen besitzen:
$X^1$ und $X^2$ stellen eine =CH-Gruppe dar oder, sofern

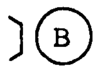

die Bedeutungen der oben genannten, zweiwertigen Reste (S), (U) oder (W) annimt, können beide oder nur $X^1$ oder nur $X^2$ auch ein Stickstoffatom darstellen;
A ist ein geradkettiger oder verzweigter gesättigter Alkylenrest mit zwei bis sieben Kohlenstoffatomen; der auch durch ein Sauerstoff- oder Schwefelatom oder durch die Methylimino- oder Ethyliminogruppe unterbrochen sein kann;
Z eine Einfachbindung, ein Sauerstoff- oder Schwefelatom, die Methylen- oder 1,2-Ethylengruppe;
$R^1$ ein verzweigter oder unverzweigter Alkylrest mit 1 bis 4 Kohlenstoffatomen oder die Benzylgruppe;
$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;
$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die $N-CH_3$-Gruppe unterbrochen sein kann;
$R^2$ kann aber auch mit A über eine Alkylenbrücke in der Weise verknüpft sein, daß zusammen mit der Gruppe $NR^1$ ein gesättigtes, 5- 6- oder 7-gliedriges heterocyclisches Ringsystem entsteht;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Wasserstoffatom, ein Fluor-, Chlor- oder Bromatom oder eine Alkylgruppe mit 1 bis 4 C-Atomen;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe, unter der Voraussetzung, daß wenn

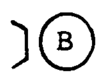

den zweiwertigen Rest (T) darstellt und $R^7$ ein Wasserstoffatom ist, $R^3$ die oben genannten Bedeutungen mit Ausnahme eines Chloratoms und Z die oben genannten Bedeutungen mit Ausnahme eines Schwefelatoms innehaben, ihre optisch aktiven Isomere und ihre physiologisch verträglichen Salze mit anorganischen oder organischen Säuren.

**2.** Kondensierte Diazepinone der allgemeinen Formel I gemäß Anspruch 1, dadurch gekennzeichnet, daß in der allgemeinen Formel I entweder
$X^1$ eine =CH-Gruppe,
$X^2$ ein Stickstoffatom und

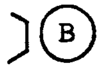

den zweiwertigen Rest (S) darstellt, mit der Maßgabe, daß $R^3$, $R^4$ und $R^5$ Wasserstoffatome sind und $R^6$ ein Wasserstoffatom, ein Chlor-oder Bromatom oder eine Methyl- oder Ethylgruppe in 8- oder 9-Stellung des Heterocyclus ist,
oder $X^1$ und $X^2$ =CH-Gruppen darstellen und

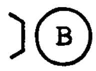

die Bedeutung des zweiwertiges Restes (U) annimmt, wobei $R^8$ ein Wasserstoffatom und $R^9$ die Methylgruppe ist, oder den Rest (V) darstellt, wobei mindestens einer der Reste $R^3$ und $R^4$ ein Wasserstoffatom ist,
A eine zwei- bis viergliedrige Alkylenkette in 3- oder 4-Stellung des gesättigten heterocyclischen Ringes,
Z die Methylengruppe und
$R^1$ und $R^2$ Alkylgruppen mit 1 bis 4 Kohlenstoffatomen oder zusammen mit dem eingeschlossenen Stickstoffatom den 1-Pyrrolidinyl-, 1-Piperidinyl-, 4-Morpholinyl- oder Hexahydro-1H-1-azepinyl-Rest bedeuten, ihre optisch aktiven Isomere und ihre physiologisch verträglichen Salze mit anorganischen oder organischen Säuren.

**3.** 6,11-Dihydro-11-[[3-[2-(1-piperidinyl)ethyl]-1-piperidinyl]carbonyl]-5H-pyrido[2,3-b][1,5]benzodiazepin-5-on und 5,11-Dihydro-11-[[3-[3-(1-piperidinyl)propyl]-1-piperidinyl]carbonyl]-6H-pyrido[2,3-b][1,4]-benzodiazepin-6-on, ihre optisch aktiven Isomere und physiologisch verträgliche Salze mit anorganischen oder organischen Säuren, gemäß Anspruch 1.

**4.** 5,11-Dihydro-11-[[4-(1-methyl-4-piperidinyl)-1-piperidinyl]-carbonyl]-6H-pyrido[2,3-b][1,4]benzodiazepin-6-on und 5,11-Dihydro-11-[[2-[2-[[1-(phenylmethyl)-4-piperidinyl]-methylamino] ethyl]-1-pyrrdidinyl]-carbonyl]-6H-pyrido[2,3-6]-[1,4]-benzodiazepin-6-on ihre optisch aktiven Isomere und physiologisch verträgliche Salze mit anorganischen oder organischen Säuren.

**5.** Arzneimittel enthaltend eine oder mehrere Verbindungen gemäß den Ansprüchen 1 bis 3 neben üblichen Träger- und/oder Hilfsstoffen.

**6.** Verwendung von Verbindungen gemäß den Ansprüchen 1 bis 3 zur Herstellung von Arzneimitteln zur Behandlung von Bradycardien und Bradyarrhythmien.

**7.** Verfahren zur Herstellung kondensierter Diazepinone der allgemeinen Formel I gemäß Anspruch I und von ihren optisch aktiven Isomeren und physiologisch verträglichen Salzen mit anorganischen oder organischen Säuren, dadurch gekennzeichnet, daß
a) zur Herstellung von basisch substituierten, kondensierten Diazepinonen der allgemeinen Formel Ia

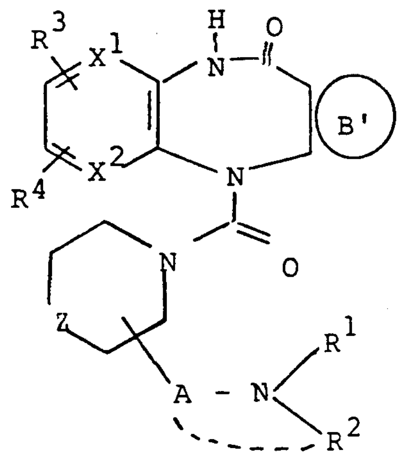

(Ia)

in der
$R^1$, $R^2$, $R^3$, $R^4$, $X^1$, $X^2$, A und Z die oben angegebenen Bedeutungen haben und

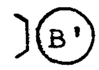

einen der zweiwertigen Reste (S), (U), (V), (W) oder (T′)

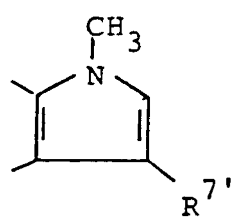

(T')

darstellt, wobei $R^{7'}$ ein Chloratom oder eine Methylgruppe ist,
Kohlensäurederivate der allgemeinen Formel II,

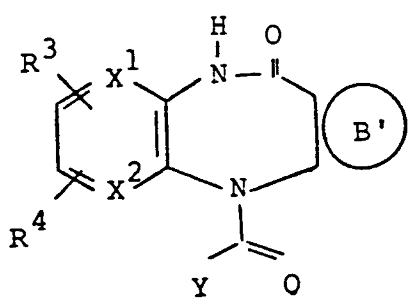

(II)

in der
$R^3$, $R^4$,

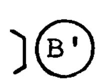 ,

$X^1$, $X^2$ die angegebenen Bedeutungen haben und
Y ein Halogenatom, bevorzugt das Brom- oder Chloratom, oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel III,

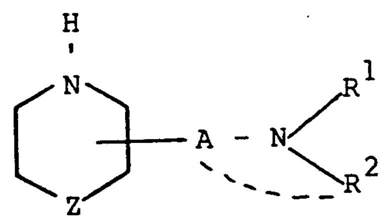

(III)

oder den entsprechenden Metallverbindungen der allgemeinen Formel IIIa

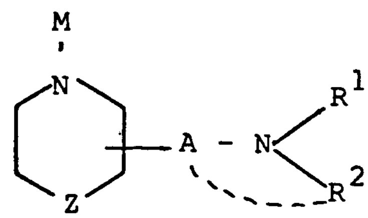

(IIIa)

wobei in den allgemeinen Formeln III und IIIa M ein Alkalimetallatom oder ein Äquivalent eines Erdalkalimetallatoms bedeutet und $R^1$, $R^2$, A und Z wie vorstehend definiert sind, bei Temperaturen zwischen -10 und +100 °C, gegebenenfalls in Gegenwart von Basen oder eines Überschußes einer Verbindung der allgemeinen Formen III und eines Lösungsmittels, umgesetzt werden, oder
b) zur Herstellung von basisch substituierten, kondensierten Diazepinonen der allgemeinen Formel Ia

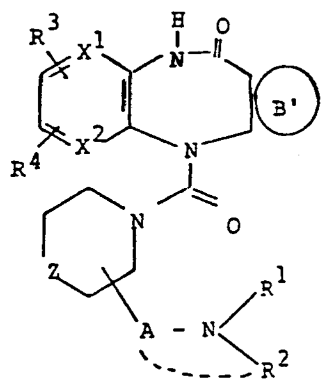

(Ia)

in der $R^1$, $R^2$, $R^3$, $R^4$, $X^1$, $X^2$, A und Z die oben angegebenen Bedeutungen haben und

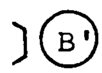

einen der zweiwertigen Reste (S), (U), (V), (W) oder (T')

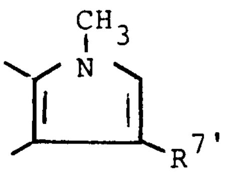

(T')

darstellt, wobei $R^{7'}$ ein Chloratom oder eine Methylgruppe ist, eine tricyclische Verbindung der allgemeinen Formel IV,

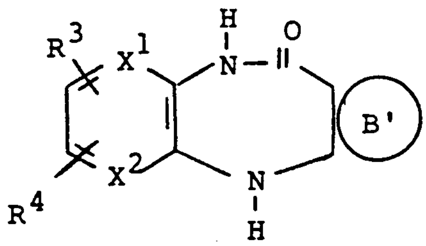

(IV)

in der die Reste $R^3$, $R^4$, $X^1$, $X^2$ und

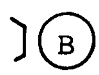

wie oben definiert sind, mit einem Chlorkohlensäurederivat der allgemeinen Formel V

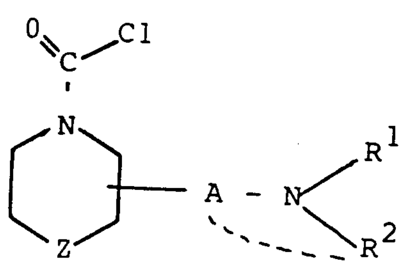

(V)

worin $R^1$, $R^2$, A und Z die oben erwähnten Bedeutungen haben, bei Temperaturen bis zum Siedepunkt des Reaktionsgemisches, gegebenenfalls in Gegenwart eines organischen Lösungsmittels und/oder einer Base umgesetzt wird, oder

c) zur Herstellung eines pyrrolo-kondensierten Diazepinons der allgemeinen Formel Ib

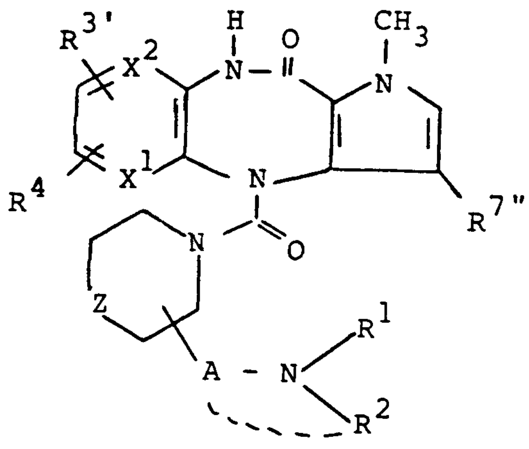

(Ib)

worin
$R^1$, $R^2$, $R^4$, $X^1$, $X^2$, A und Z die eingangs erwähnten Bedeutungen, für Z mit Ausnahme der eines Schwefelatoms, haben, $R^{7''}$ ein Wasserstoffatom und $R^{3'}$ eine Alkylgruppe mit 1 bis 4 C-Atomen oder ein Wasserstoffatom darstellen, eine Verbindung der allgemeinen Formel Ib, in der $R^7$ ein Chloratom bedeutet, hydrogenolysiert wird entweder mit Wasserstoff in Gegenwart von Katalysatoren und Lösungsmitteln bei Temperaturen zwischen 0 und 130 ° C mit Wasserstoffdrucken von 1 bis 300 bar oder mit Ameisensäure in Gegenwart von Lösungsmitteln und Palladium oder Palladiumacetat als Katalysator bei Temperaturen zwischen 40 und 110 ° C,
und gegebenenfalls, erhaltene Verbindungen der allgemeinen Formel I in ihre Säureadditionssalze mit anorganischen oder organischen Säuren, oder erhaltene Säureadditionssalze in die freien Basen übergeführt werden und/oder erhaltene Racemate, gewünschtenfalls in ihre Isomeren mittels optisch aktiver Säuren aufgetrennt werden.

**8.** Verfahren gemäß Anspruch 6a, dadurch gekennzeichnet, daß die Umsetzung in Gegenwart aprotischer polarer Lösungsmittel bei Temperaturen zwischen 40 und 100 ° C in Gegenwart von Alkali- oder Erdalkalihydroxiden, -alkoholaten oder -carbonaten oder von tertiären Aminen durchgeführt wird.

**9.** Verfahren gemäß Anspruch 6b, dadurch gekennzeichnet, daß die Umsetzung in Gegenwart von aromatischen Kohlenwasserstoffen, in Äthern, in Ketonen, in chlorierten aliphatischen Kohlenwasserstoffen, in Acetonitil oder Dimethylformamid oder in Gemischen dieser Lösungsmittel und in Gegenwart von tertiären Aminen bei Temperaturen zwischen 30 und 100 ° C durchgeführt wird.

**10.** Verfahren gemäß Anspruch 6c, dadurch gekennzeichnet, daß die Hydrogenolyse in Gegenwart von Metallen der VIII. Nebengruppe des Periodensystems der Elemente, gegebenenfalls in Gegenwart von Chlorwasserstoff-Akzeptoren oder mit Hilfe von Ameisensäure in Gegenwart von Dimethylformamid und Palladium auf Kohle oder mit Hilfe von Triethylammoniumformiat in Gegenwart überschüssigen Triethylamins und Palladium auf Tierkohle oder von Palladiumacetat und Triarylphosphinen bei Temperaturen zwischen 40 und 110 ° C durchgeführt wird.

**Patentansprüche für folgende Vertragsstaaten : ES, GR**

**1.** Verfahren zur Herstellung kondensierter Diazepinone der allgemeinen Formel I

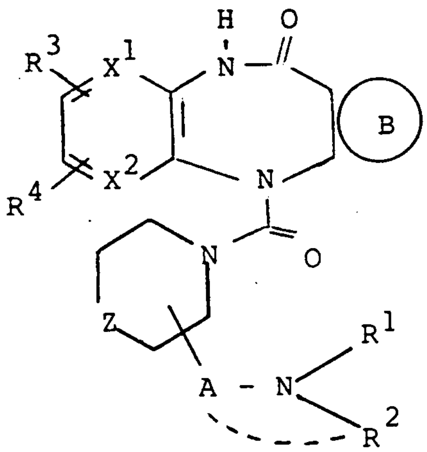

(I)

in der

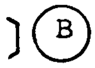

einen der zweiwertigen Reste

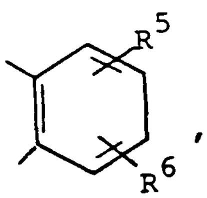 , 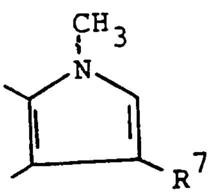 , 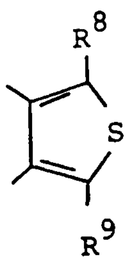 , 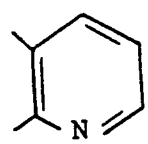 oder 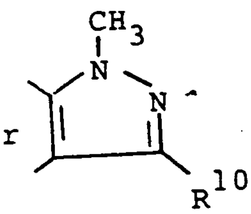

(S) (T) (U) (V) (W)

darstellt und
$X^1$, $X^2$, A, $R^1$ bis $R^{10}$ und Z die folgenden Bedeutungen besitzen:
$X^1$ und $X^2$ stellen eine =CH-Gruppe dar oder, sofern

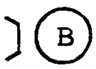

die Bedeutungen der oben genannten, zweiwertigen Reste (S), (U) oder (W) annimmt, können beide oder nur $X^1$ oder nur $X^2$ auch ein Stickstoffatom darstellen;
A ist ein geradkettiger oder verzweigter gesättigter Alkylenrest mit zwei bis sieben Kohlenstoffatomen; der auch durch ein Sauerstoff- oder Schwefelatom oder durch die Methylimino- oder Ethyliminogruppe unterbrochen sein kann;
Z eine Einfachbindung, ein Sauerstoff- oder Schwefelatom, die Methylen- oder 1,2-Ethylengruppe;
$R^1$ ein verzweigter oder unverzweigter Alkylrest mit 1 bis 4 Kohlenstoffatomen oder die Benzylgruppe;
$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;
$R^1$ und $R^2$ können aber auch zusammen mit dem dazwischenliegenden Stickstoffatom einen 4- bis 7-

gliedrigen gesättigten, monocyclischen, heterocyclischen Ring bilden, der gegebenenfalls durch ein Sauerstoffatom oder durch die N-$CH_3$-Gruppe unterbrochen sein kann;
$R^2$ kann aber auch mit A über eine Alkylenbrücke in der Weise verknüpft sein, daß zusammen mit der Gruppe $NR^1$ ein gesättigtes, 5-, 6- oder 7-gliedriges heterocyclisches Ringsystem entsteht;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Wasserstoffatom, ein Fluor-, Chlor- oder Bromatom oder eine Alkylgruppe mit 1 bis 4 C-Atomen;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe, unter der Voraussetzung, daß wenn

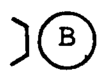

den zweiwertigen Rest (T) darstellt und $R^7$ ein Wasserstoffatom ist, $R^3$ die oben genannten Bedeutungen mit Ausnahme eines Chloratoms und Z die oben genannten Bedeutungen mit Ausnahme eines Schwefelatoms innehaben und von ihren optisch aktiven Isomeren und physiologisch verträglichen Salzen mit anorganischen oder organischen Säuren, dadurch gekennzeichnet, daß

a) zur Herstellung von basisch substituierten, kondensierten Diazepinonen der allgemeinen Formel Ia

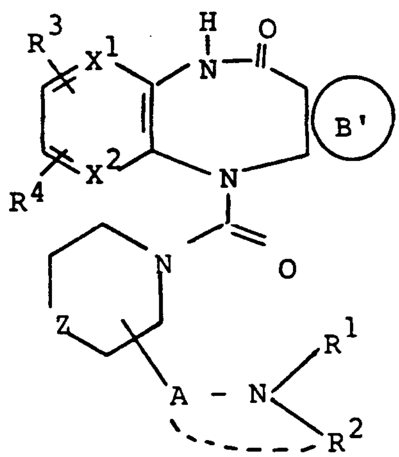

(Ia)

in der
$R^1$, $R^2$, $R^3$, $R^4$, $X^1$, $X^2$, A und Z die oben angegebenen Bedeutungen haben und

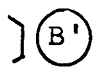

einen der zweiwertigen Reste (S), (U), (V), (W) oder (T,)

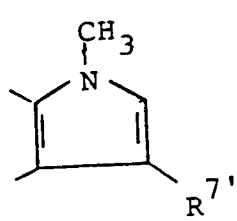

(T')

darstellt, wobei $R^{7'}$ ein Chloratom oder eine Methylgruppe ist,

Kohlensäurederivate der allgemeinen Formel II,

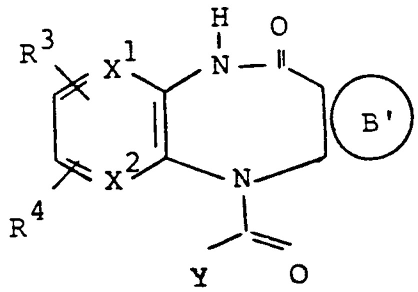

(II)

in der
$R^3$, $R^4$,

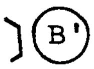

,

$X^1$, $X^2$ die angegebenen Bedeutungen haben und
Y ein Halogenatom, bevorzugt das Brom- oder Chloratom, oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel III,

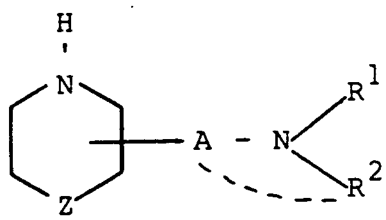

(III)

oder den entsprechenden Metallverbindungen der allgemeinen Formel IIIa

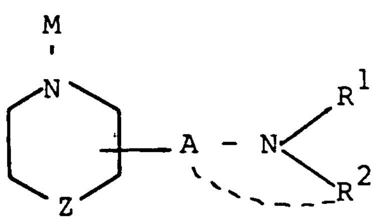

(IIIa)

wobei in den allgemeinen Formeln III und IIIa M ein Alkalimetallatom oder ein Äquivalent eines Erdalkalimetallatoms bedeutet und $R^1$, $R^2$, A und Z wie vorstehend definiert sind, bei Temperaturen zwischen -10 und $+100\,^\circ C$, gegebenenfalls in Gegenwart von Basen oder eines Überschußes einer Verbindung der allgemeinen Formen III und eines Lösungsmittels, umgesetzt werden, oder
b) zur Herstellung von basisch substituierten, kondensierten Diazepinonen der allgemeinen Formel Ia

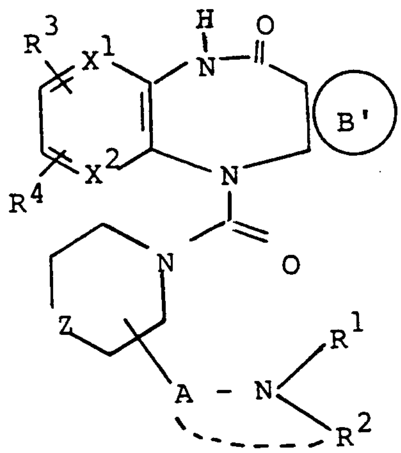

(Ia)

in der $R^1$, $R^2$, $R^3$, $R^4$, $X^1$, $X^2$, A und Z die oben angegebenen Bedeutungen haben und

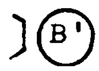

einen der zweiwertigen Reste (S), (U), (V), (W) oder (T′)

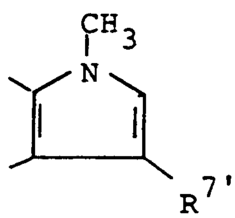

(T')

darstellt, wobei $R^{7'}$ ein Chloratom oder eine Methylgruppe ist, eine tricyclische Verbindung der allgemeinen Formel IV,

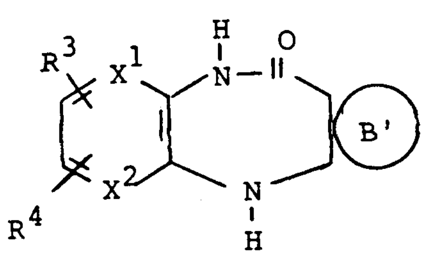

(IV)

in der die Reste $R^3$, $R^4$, $X^1$, $X^2$ und

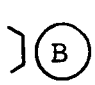

wie oben definiert sind, mit einem Chlorkohlensäurederivat der allgemeinen Formel V

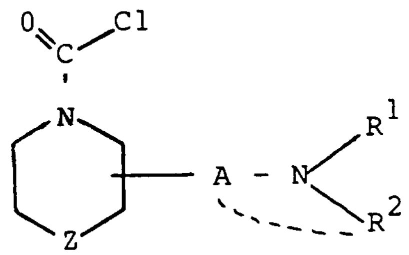

(V)

worin $R^1$, $R^2$, A und Z die oben erwähnten Bedeutungen haben, bei Temperaturen bis zum Siedepunkt des Reaktionsgemisches, gegebenenfalls in Gegenwart eines organischen Lösungsmittels und/oder einer Base umgesetzt wird, oder

c) zur Herstellung eines pyrrolo-kondensierten Diazepinons der allgemeinen Formel Ib

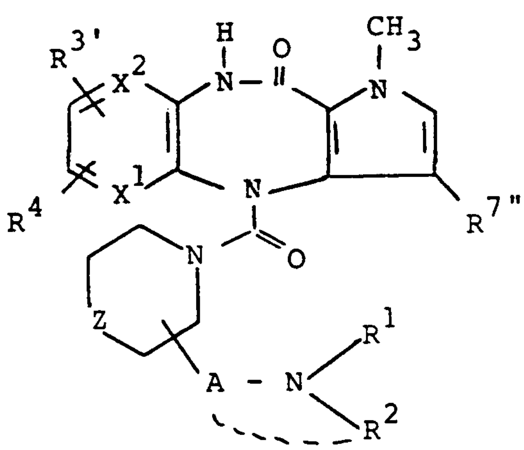

(Ib)

worin

$R^1$, $R^2$, $R^4$, $X^1$, $X^2$, A und Z die eingangs erwähnten Bedeutungen, für Z mit Ausnahme der eines Schwefelatoms, haben, $R^{7''}$ ein Wasserstoffatom und $R^{3'}$ eine Alkylgruppe mit 1 bis 4 C-Atomen oder ein Wasserstoffatom darstellen, eine Verbindung der allgemeinen Formel Ib, in der $R^7$ ein Chloratom bedeutet, hydrogenolysiert wird entweder mit Wasserstoff in Gegenwart von Katalysatoren und Lösungsmitteln bei Temperaturen zwischen 0 und 130 °C mit Wasserstoffdrucken von 1 bis 300 bar oder mit Ameisensäure in Gegenwart von Lösungsmitteln und Palladium oder Palladiumacetat als Katalysator bei Temperaturen zwischen 40 und 110 °C,

und gegebenenfalls, erhaltene Verbindungen der allgemeinen Formel I in ihre Säureadditionssalze mit anorganischen oder organischen Säuren, oder erhaltene Säureadditionssalze in die freien Basen übergeführt werden und/oder erhaltene Racemate, gewünschtenfalls in ihre Isomeren mittels optisch aktiver Säuren aufgetrennt werden.

**2.** Verfahren gemäß Anspruch 1a, dadurch gekennzeichnet, daß die Umsetzung in Gegenwart aprotischer polarer Lösungsmittel bei Temperaturen zwischen 40 und 100 °C in Gegenwart von Alkali- oder Erdalkalihydroxiden, -alkoholaten oder -carbonaten oder von tertiären Aminen durchgeführt wird.

**3.** Verfahren gemäß Anspruch 1b, dadurch gekennzeichnet, daß die Umsetzung in Gegenwart von aromatischen Kohlenwasserstoffen, in Äthern, in Ketonen, in chlorierten aliphatischen Kohlenwasserstoffen, in Acetonitil oder Dimethylformamid oder in Gemischen dieser Lösungsmittel und in Gegenwart von tertiären Aminen bei Temperaturen zwischen 30 und 100 °C durchgeführt wird.

**4.** Verfahren gemäß Anspruch 1c, dadurch gekennzeichnet, daß die Hydrogenolyse in Gegenwart von Metallen der VIII. Nebengruppe des Periodensystems der Elemente, gegebenenfalls in Gegenwart von Chlorwasserstoff-Akzeptoren oder mit Hilfe von Ameisensäure in Gegenwart von Dimethylformamid und Palladium auf Kohle oder mit Hilfe von Triethylammoniumformiat in Gegenwart überschüssigen Triethylamins und Palladium auf Tierkohle oder von Palladiumacetat und Triarylphosphinen bei Temperaturen zwischen 40 und 110 °C durchgeführt wird.

## Claims

**Claims for the following Contracting States : AT, BE, CH, DE, FR, GB, IT, LI, LU, NL, SE**

1. Condensed diazepinones of general formula I

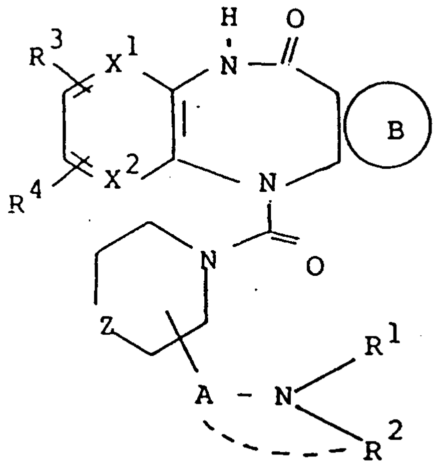

(I)

wherein

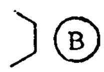

represents one of the divalent groups

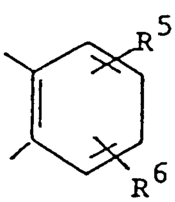 ,
(S)

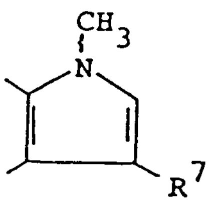 ,
(T)

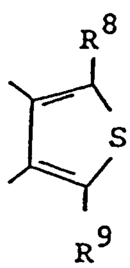 ,
(U)

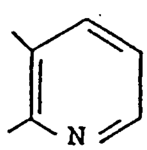 or
(V)

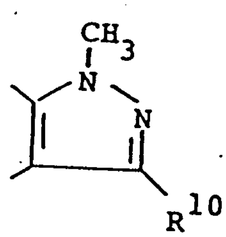
(W)

and
$X^1$, $X^2$, A, $R^1$ to $R^{10}$ and Z are defined as follows:
$X^1$ and $X^2$ represent a =CH- group or, if

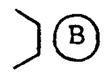

represents the above-mentioned divalent groups (S), (U) or (W), both $X^1$ and $X^2$ or only $X^1$ or only $X^2$ may represent a nitrogen atom;
A is a straight-chained or branched saturated alkylene group with two to seven carbon atoms which may also be interrupted by an oxygen or sulphur atom or by a methylimino or ethylimino group;
Z represents a single bond, an oxygen or sulphur atom or a methylene or 1,2-ethylene group;
$R^1$ represents a branched or unbranched alkyl group with 1 to 4 carbon atoms or a benzyl group;

$R^2$ represents a branched or unbranched alkyl group with 1 to 7 carbon atoms which may optionally also be substituted by a hydroxy group at its 2nd to 7th carbon atom, a cycloalkyl or cycloalkylmethyl group with 3 to 7 carbon atoms in the ring, wherein the cycloalkyl ring may optionally also be substituted by a hydroxy group;
$R^1$ and $R^2$ may, however, also form, together with the intermediate nitrogen atom, a 4- to 7-membered saturated, monocyclic, heterocyclic ring which may optionally be interrupted by an oxygen atom or by an $N\text{-}CH_3$ group;
$R^2$ may, however, also be linked to A via an alkylene bridge so that, in conjunction with the group $NR^1$, a saturated 5-, 6- or 7-membered heterocyclic ring system is produced;
$R^3$ is an alkyl group with 1 to 4 carbon atoms, a chlorine atom or a hydrogen atom;
$R^4$ is a hydrogen atom or a methyl group;
$R^5$ and $R^6$ each represent a hydrogen atom, a fluorine, chlorine or bromine atom or an alkyl group with 1 to 4 carbon atoms;
$R^7$ represents a hydrogen or chlorine atom or a methyl group;
$R^8$ represents a hydrogen atom or an alkyl group with 1 to 4 carbon atoms;
$R^9$ represents a hydrogen or halogen atom or an alkyl group with 1 to 4 carbon atoms and
$R^{10}$ represents a hydrogen atom or a methyl group, with the proviso that if

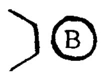

represents the divalent (T) and $R^7$ is a hydrogen atom, $R^3$ has the meanings given hereinbefore with the exception of a chlorine atom and Z has the meanings given hereinbefore with the exception of a sulphur atom,
the optically active isomers thereof and the physiologically acceptable salts thereof with inorganic or organic acids.

**2.** Condensed diazepinones of general formula I as claimed in claim 1, characterised in that, in general formula I either
$X^1$ is a =CH- group,
$X^2$ represents a nitrogen atom and

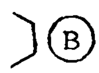

represents a divalent group (S), with the proviso that $R^3$,
$R^4$ and $R^5$ are hydrogen atoms and $R^6$ is a hydrogen atom or a chlorine or bromine atom or a methyl or ethyl group in the 8 or 9 position of the heterocycle,
or $X^1$ and 2 represent =CH- groups and

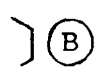

represents the divalent group (U), wherein $R^6$ is a hydrogen atom and $R^9$ is a methyl group, or the group (V), wherein at least one of the groups $R^3$ and $R^4$ is a hydrogen atom,
A is a two- to four- membered alkylene chain in the 3 or 4 position of the saturated heterocyclic ring,
Z is a methylene group and
$R^1$ and $R^2$ represent alkyl groups with 1 to 4 carbon atoms or together with the intermediate nitrogen atom represent the 1-pyrrolidinyl, 1-piperidinyl, 4-morpholinyl or hexahydro-1H-1-azepinyl group,
the optically active isomers and physiologically acceptable salts thereof with inorganic or organic acids.

**3.** 6,11-Dihydro-11-[[3-[2-(1-piperidinyl)ethyl]-1-piperidinyl]carbonyl]-5H-pyrido[2,3-b][1,5]benzodiazepin-5-one and 5,11-dihydro-11-[[3-[3-(1-piperidinyl)propyl]-1-piperidinyl]carbonyl]-6H-piprido[2,3-b][1,4]-benzodiazepin-6-one, the optically active isomers and physiologically acceptable salts thereof with inorganic or organic acids, according to claim 1.

4. 5,11-Dihydro-11-[[4-(1-methyl-4-piperidinyl)-1-piperidinyl]-carbonyl]-6H-pyrido[2,3-b][1,4]benzodiazepin-6-one and 5,11-dihydro-11-[[2-[2-[[1-(phenylmethyl)-4-piperidinyl]-methyl-amino]-ethyl]-1-pyrrolidinyl]-carbonyl]-6H-pyrido[2,3-b][1,4]benzodiazepin-6-one, the optically active isomers and physiologically acceptable salts thereof with organic and inorganic acids.

5. Pharmaceutical compositions containing one or more compounds as claimed in claims 1 to 3 together with conventional carriers and/or excipients.

6. Use of compounds as claimed in claims 1 to 3 for the preparation of pharmaceutical compositions for the treatment of bradycardia and bradyarrhythmia.

7. Process for preparing condensed diazepinones of general formula I according to claim 1 and the optically active isomers and physiologically acceptable salts thereof with organic and inorganic acids, characterised in that

a) to prepare base substituted condensed diazepinones of general formula Ia

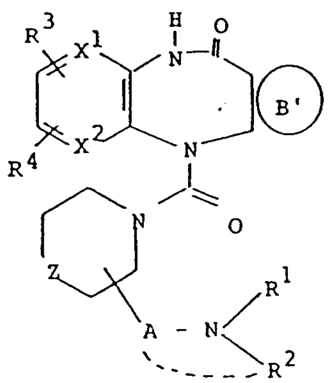

(Ia)

wherein

$R^1$, $R^2$, $R^3$, $R^4$, $X^1$, $X^2$, A and Z are as hereinbefore defined and

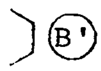

represents one of the divalent groups (S), (U), (V), (W) or (T')

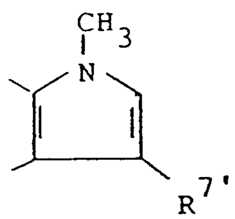

(T')

wherein $R^{7'}$ is a chlorine atom or a methyl group,

carbonic acid derivatives of general formula II

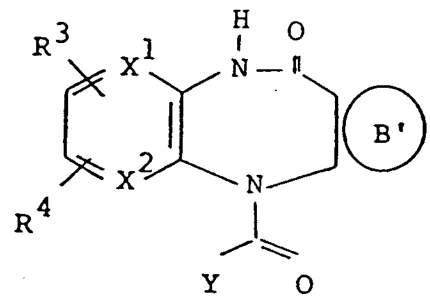

(II)

wherein
$R^3$, $R^4$,

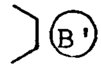

,

$X^1$ and $X^2$ are as hereinbefore defined and
Y represents a halogen atom, preferably a bromine or chlorine atom, or the group $OR^{11}$, wherein $R^{11}$ represents an optionally halogen-substituted alkyl group with 1 to 5 carbon atoms, a phenyl group optionally substituted by halogen atoms or nitro groups or an aralkyl group with 7 to 15 carbon atoms, are reacted
with compounds of general formula III

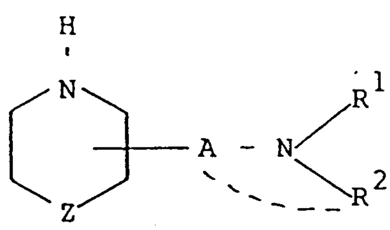

(III)

or the corresponding metal compounds of general formula IIIa

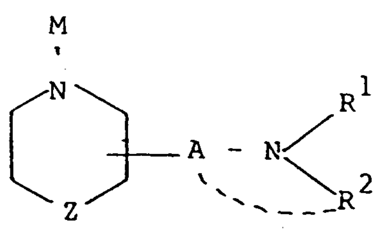

(IIIa)

wherein, in general formulae III and IIIa,
M represents an alkali metal atom or one equivalent of an alkaline earth metal atom, and $R^1$, $R^2$, A and Z are as hereinbefore defined, at temperatures between -10 and +100°C, optionally in the presence of bases or an excess of a compound of general formula III and a solvent, or
b) in order to prepare base substituted, condensed diazepinones of general formula Ia

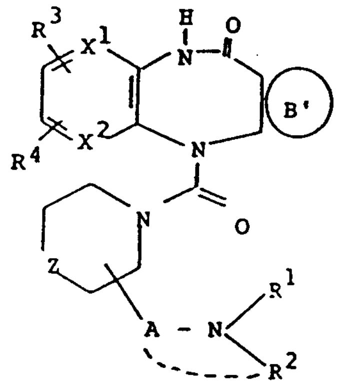

(Ia)

wherein $R^1$, $R^2$, $R^3$, $R^4$, $X^1$, $X^2$, A and Z are as hereinbefore defined and

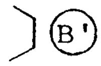

represents one of the divalent groups (S), (U), (V), (W) or (T')

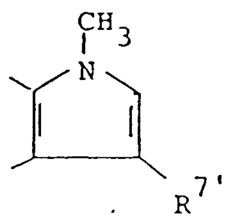

(T')

wherein $R^{7'}$ is a chlorine atom or a methyl group,
a tricyclic compound of general formula IV

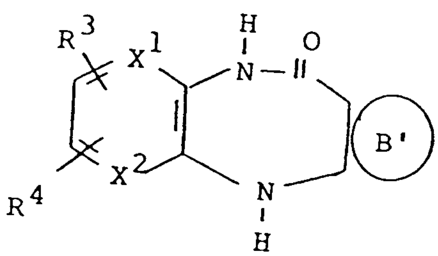

(IV)

wherein the groups $R^3$, $R^4$, $X^1$, $X^2$ and

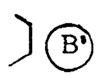

are as hereinbefore defined, is reacted with a chlorocarbonic acid derivative of general formula V

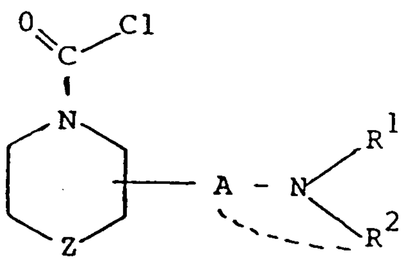

(V)

wherein $R^1$, $R^2$, A and Z are as hereinbefore defined, at temperatures up to the boiling point of the reaction mixture, optionally in the presence of an organic solvent and/or a base, or
c) in order to prepare a pyrrolo-condensed diazepinone of general formula Ib

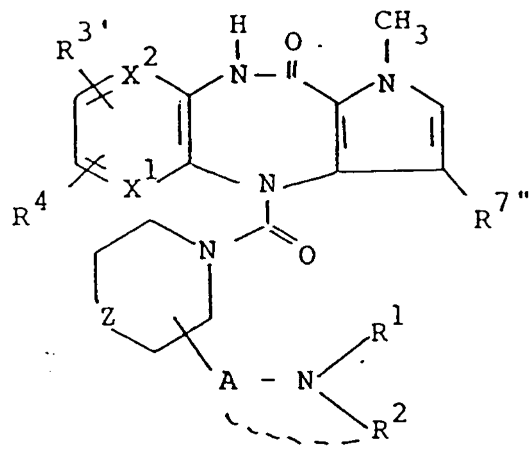

(Ib)

wherein
$R^1$, $R^2$, $R^4$, $X^1$, $X^2$, A and Z have the meanings as hereinbefore defined, with the exception for Z of a sulphur atom, $R^{7''}$ represents a hydrogen atom and $R^{3'}$ represents an alkyl group with 1 to 4 carbon atoms or a hydrogen atom, a compound of general formula Ib wherein $R^7$ represents a chlorine-atom is hydrogenolysed either with hydrogen in the presence of catalysts and solvents at temperatures of between 0 and 130 °C under hydrogen pressures of 1 to 300 bar or with formic acid in the presence of solvents and palladium or palladium acetate as catalyst at temperatures of between 40 and 110 °C,
and optionally, if compounds of general formula I are obtained they are converted into the acid addition salts thereof with inorganic or organic acids, or if acid addition salts are obtained they are converted into the free bases and/or if racemates are obtained they are if desired separated into their isomers using optically active acids.

**8.** Process as claimed in claim 7a, characterised in that the reaction is carried out in the presence of aprotic polar solvents at temperatures of between 40 and 100 °C in the presence of alkali or alkaline earth metal hydroxides, alkoxides or carbonates or tertiary amines.

**9.** Process as claimed in claim 7b, characterised in that the reaction is carried out in the presence of aromatic hydrocarbons, in ethers, in ketones, in chlorinated aliphatic hydrocarbons, in acetonitrile or dimethylformamide or in mixtures of these solvents and in the presence of tertiary amines at temperatures of betwen 30 and 100 °C.

**10.** Process as claimed in claim 7c, characterised in that the hydrogenolysis is carried out in the presence of metals of the VIIIth sub-group of the periodic table of elements, optionally in the presence of hydrogen chloride acceptors or using formic acid in the presence of dimethylformamide and palladium on charcoal or using triethylammonium formate in the presence of excess triethylamine and palladium on animal charcoal or palladium acetate and triarylphosphines at temperatures of between 40 and 110 °C.

**Claims for the following Contracting States : ES, GR**

**1.** Process for preparing condensed diazepinones of general formula I

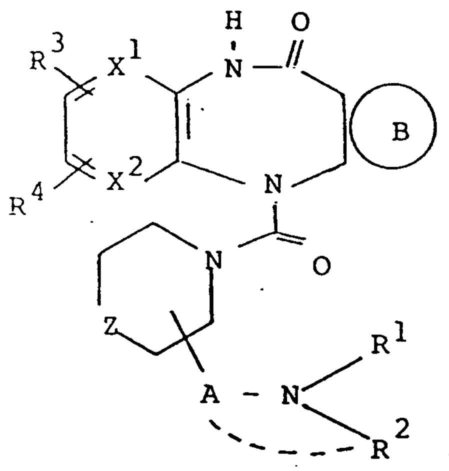

(I)

wherein

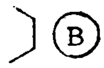

represents one of the divalent groups

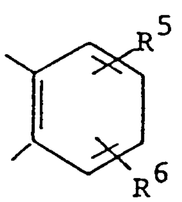 , 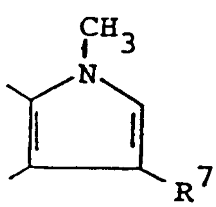 , 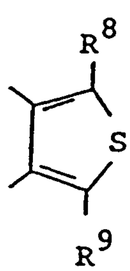 , 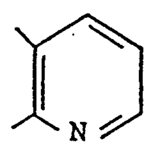 or 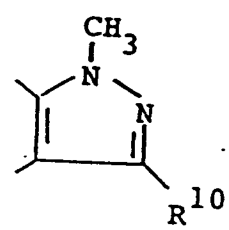

(S) (T) (U) (V) (W)

and
$X^1$, $X^2$, A, $R^1$ to $R^{10}$ and Z are defined as follows:
$X^1$ and $X^2$ represent a =CH- group or, if

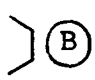

represents the above-mentioned divalent groups (S), (U) or (W), both $X^1$ and $X^2$ or only $X^1$ or only $X^2$ may represent a nitrogen atom;
A is a straight-chained or branched saturated alkylene group with two to seven carbon atoms which may also be interrupted by an oxygen or sulphur atom or by a methylimino or ethylimino group;
Z represents a single bond, an oxygen or sulphur atom or a methylene or 1,2-ethylene group;
$R^1$ represents a branched or unbranched alkyl group with 1 to 4 carbon atoms or a benzyl group;
$R^2$ represents a branched or unbranched alkyl group with 1 to 7 carbon atoms which may optionally also be substituted by a hydroxy group at its 2nd to 7th carbon atom, a cycloalkyl or cycloalkylmethyl group with 3 to 7 carbon atoms in the ring, wherein the cycloalkyl ring may optionally also be substituted by a hydroxy group;
$R^1$ and $R^2$ may, however, also form, together with the intermediate nitrogen atom, a 4- to 7-membered

saturated, monocyclic, heterocyclic ring which may optionally be interrupted by an oxygen atom or by an N-$CH_3$ group;
$R^2$ may, however, also be linked to A via an alkylene bridge so that, in conjunction with the group $NR^1$, a saturated 5-, 6- or 7-membered heterocyclic ring system is produced;
$R^3$ is an alkyl group with 1 to 4 carbon atoms, a chlorine atom or a hydrogen atom;
$R^4$ is a hydrogen atom or a methyl group;
$R^5$ and $R^6$ each represent a hydrogen atom, a fluorine, chlorine or bromine atom or an alkyl group with 1 to 4 carbon atoms;
$R^7$ represents a hydrogen or chlorine atom or a methyl group;
$R^8$ represents a hydrogen atom or an alkyl group with 1 to 4 carbon atoms;
$R^9$ represents a hydrogen or halogen atom or an alkyl group with 1 to 4 carbon atoms and
$R^{10}$ represents a hydrogen atom or a methyl group, with the proviso that if

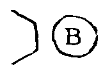

represents the divalent (T) and $R^7$ is a hydrogen atom, $R^3$ has the meanings given hereinbefore with the exception of a chlorine atom and Z has the meanings given hereinbefore with the exception of a sulphur atom,
the optically active isomers thereof and the physiologically acceptable salts thereof with inorganic or organic acids, characterised in that

a) to prepare base substituted condensed diazepinones of general formula Ia

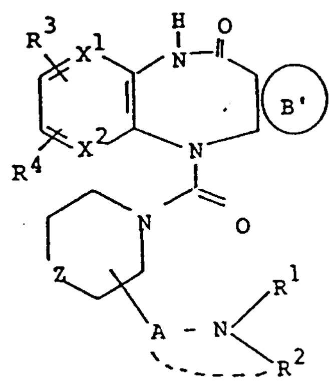

(Ia)

wherein
$R^1$, $R^2$, $R^3$, $R^4$, $X^1$, $X^2$, A and Z are as hereinbefore defined and

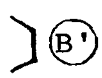

represents one of the divalent groups (S), (U), (V), (W) or (T')

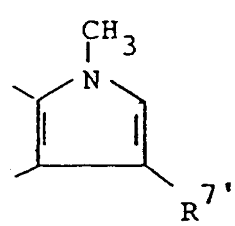

(T')

wherein $R^{7'}$ is a chlorine atom or a methyl group,
carbonic acid derivatives of general formula II

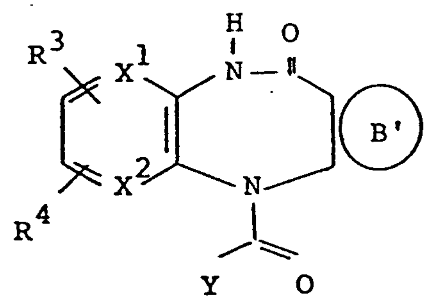

(II)

wherein

$R^3$, $R^4$,

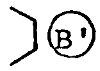

,

$X^1$ and $X^2$ are as hereinbefore defined and

Y represents a halogen atom, preferably a bromine or chlorine atom, or the group $OR^{11}$, wherein $R^{11}$ represents an optionally halogen-substituted alkyl group with 1 to 5 carbon atoms, a phenyl group optionally substituted by halogen atoms or nitro groups or an aralkyl group with 7 to 15 carbon atoms, are reacted

with compounds of general formula III

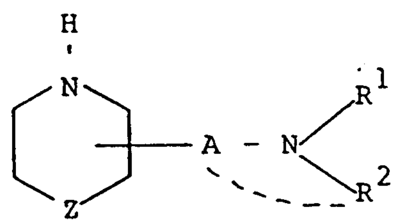

(III)

or the corresponding metal compounds of general formula IIIa

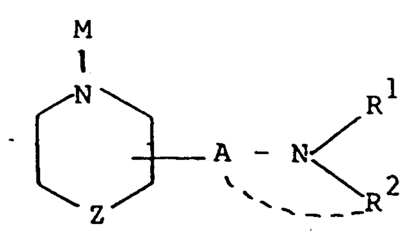

(IIIa)

wherein, in general formulae III and IIIa,

M represents an alkali metal atom or one equivalent of an alkaline earth metal atom, and $R^1$, $R^2$, A and Z are as hereinbefore defined, at temperatures between -10 and +100 °C, optionally in the presence of bases or an excess of a compound of general formula III and a solvent, or

b) in order to prepare base substituted, condensed diazepinones of general formula Ia

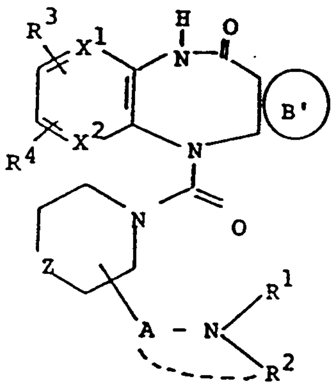

(Ia)

wherein $R^1$, $R^2$, $R^3$, $R^4$, $X^1$, $X^2$, A and Z are as hereinbefore defined and

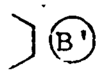

represents one of the divalent groups (S), (U), (V), (W) or (T')

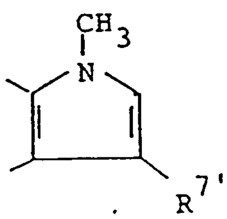

(T')

wherein $R^{7'}$ is a chlorine atom or a methyl group,
a tricyclic compound of general formula IV

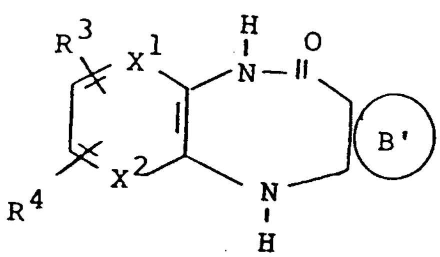

(IV)

wherein the groups $R^3$, $R^4$, $X^1$, $X^2$ and

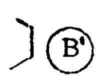

are as hereinbefore defined, is reacted with a chlorocarbonic acid derivative of general formula V

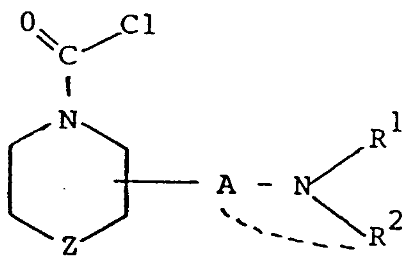

(V)

wherein $R^1$, $R^2$, A and Z are as hereinbefore defined, at temperatures up to the boiling point of the reaction mixture, optionally in the presence of an organic solvent and/or a base, or
c) in order to prepare a pyrrolo-condensed diazepinone of general formula Ib

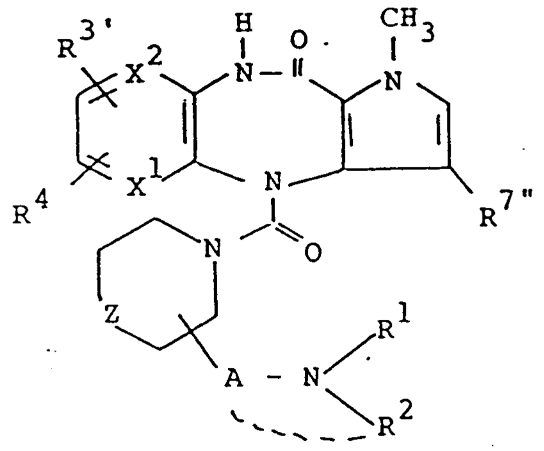

(Ib)

wherein
$R^1$, $R^2$, $R^4$, $X^1$, $X^2$, A and Z have the meanings as hereinbefore defined, with the exception for Z of a sulphur atom, $R^{7''}$ represents a hydrogen atom and $R^{3'}$ represents an alkyl group with 1 to 4 carbon atoms or a hydrogen atom, a compound of general formula Ib wherein $R^7$ represents a chlorine atom is hydrogenolysed either with hydrogen in the presence of catalysts and solvents at temperatures of between 0 and 130 °C under hydrogen pressures of 1 to 300 bar or with formic acid in the presence of solvents and palladium or palladium acetate as catalyst at temperatures of between 40 and 110 °C,
and optionally, if compounds of general formula I are obtained they are converted into the acid addition salts thereof with inorganic or organic acids, or if acid addition salts are obtained they are converted into the free bases and/or if racemates are obtained they are if desired separated into their isomers using optically active acids.

**2.** Process as claimed in claim 1a, characterised in that the reaction is carried out in the presence of aprotic polar solvents at temperatures of between 40 and 100 °C in the presence of alkali or alkaline earth metal hydroxides, alkoxides or carbonates or tertiary amines.

**3.** Process as claimed in claim 1b, characterised in that the reaction is carried out in the presence of aromatic hydrocarbons, in ethers, in ketones, in chlorinated aliphatic hydrocarbons, in acetonitrile or dimethylformamide or in mixtures of these solvents and in the presence of tertiary amines at temperatures of betwen 30 and 100 °C.

**4.** Process as claimed in claim 1c, characterised in that the hydrogenolysis is carried out in the presence of metals of the VIIIth sub-group of the periodic table of elements, optionally in the presence of hydrogen chloride acceptors or using formic acid in the presence of dimethylformamide and palladium on charcoal or using triethylammonium formate in the presence of excess triethylamine and palladium on animal charcoal or palladium acetate and triarylphosphines at temperatures of between 40 and 110 °C.

## Revendications

### Revendications pour les Etats contractants suivants : AT, BE, CH, DE, FR, GB, IT, LI, LU, NL, SE

**1.** Diazépinones condensées de formule générale I

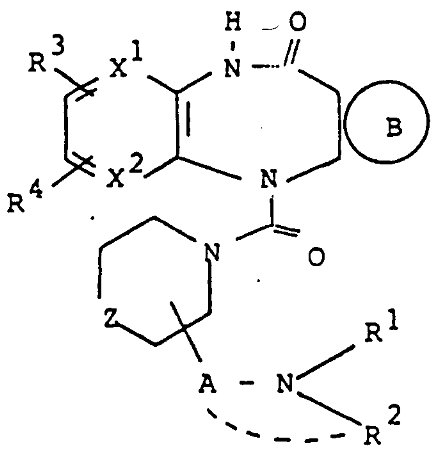

(I)

dans laquelle

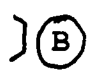

représente l'un des restes divalents

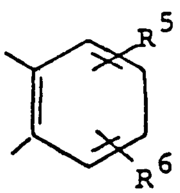

(S)

,

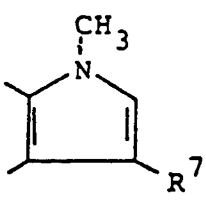

(T)

,

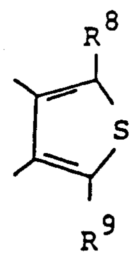

(U)

,

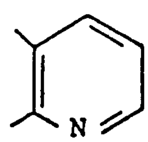

(V)

ou

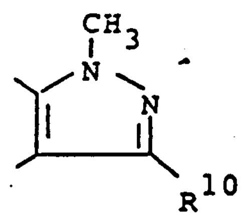

(W)

et
$X^1$, $X^2$, A, $R^1$ à $R^{10}$ et Z ont les significations suivantes:
$X^1$ et $X^2$ représentent un groupe =CH ou, lorsque

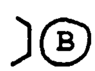

revêt les significations des restes divalents (S), (U) ou (W) cités ci-dessus, peuvent aussi représenter tous les deux ou seulement $X^1$ ou seulement $X^2$ un atome d'azote;
A est un reste alkylène saturé linéaire ou ramifié ayant deux à sept atomes de carbone; qui peut aussi être interrompu par un atome d'oxygène ou de soufre ou par le groupe méthylimino ou éthylimino;
Z est une liaison simple, un atome d'oxygène ou de soufre, le groupe méthylène ou 1,2-éthylène;
$R^1$ est un reste alkyle ramifié ou non ramifié ayant 1 à 4 atomes de carbone ou le groupe benzyle;
$R^2$ est un reste alkyle ramifié ou non ramifié ayant 1 à 7 atomes de carbone qui peut encore être substitué éventuellement sur son deuxième à septième atome de carbone par un groupe hydroxyle, un reste cycloalkyle ou cycloalkylméthyle ayant 3 à 7 atomes de carbone dans le cycle, la cycle cycloalkyle pouvant éventuellement être substitué encore par un groupe hydroxyle;

$R^1$ et $R^2$ peuvent cependant former aussi avec l'atome d'azote situé entre eux un cycle hétérocyclique saturé, monocyclique, ayant 4 à 7 chaînons qui peut éventuellement être interrompu par un atome d'oxygène ou par le groupe N-$CH_3$;
$R^2$ peut aussi être relié à A par un pont alkylène pour former avec le groupe $NR^1$ un système cyclique hétérocyclique saturé à 5, 6 ou 7 chaînons;
$R^3$ est un groupe alkyle ayant 1 à 4 atomes de carbone, un atome de chlore ou un atome d'hydrogène;
$R^4$ est un atome d'hydrogène ou un groupe méthyle;
$R^5$ et $R^6$ représentent chacun un atome d'hydrogène, un atome de fluor, de chlore ou de brome ou un groupe alkyle ayant 1 à 4 atomes de carbone;
$R^7$ est un atome d'hydrogène ou de chlore ou un groupe méthyle;
$R^8$ est un atome d'hydrogène ou un reste alkyle ayant 1 à 4 atomes de carbone;
$R^9$ est un atome d'hydrogène ou un atome d'halogène ou un groupe alkyle ayant 1 à 4 atomes de carbone; et
$R^{10}$ est un atome d'hydrogène ou un groupe méthyle, à condition que lorsque

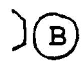

représente le reste divalent (T) et $R^7$ est un atome d'hydrogène, $R^3$ ait les significations indiquées ci-dessus à l'exception d'un atome de chlore et Z ait les significations indiquées ci-dessus à l'exception d'un atome de soufre, leurs isomères optiquement actifs et leurs sels, compatibles du point de vue physiologique, avec des acides inorganiques et organiques.

**2.** Diazépinones condensées de formule générale I selon la revendication 1, caractérisées en ce que dans la formule générale I
$X^1$ représente un groupe =CH,
$X^2$ représente un atome d'azote et

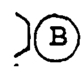

représente le reste divalent (S), à condition que $R^3$, $R^4$ et $R^5$ soient des atomes d'hydrogène et que $R^6$ soit un atome d'hydrogène, un atome de chlore ou un atome de brome ou un groupe méthyle ou éthyle en huitième ou neuvième position de l'hétérocycle,
ou bien $X^1$ et $X^2$ représentent des groupes =CH et

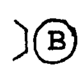

revêt la signification du reste divalent (U), $R^8$ étant un atome d'hydrogène et $R^9$ un groupe méthyle, ou représentant le reste (V), au moins l'un des restes $R^3$ et $R^4$ étant un atome d'hydrogène,
A représente une chaîne alkylène ayant deux à quatre chaînons en position 3 ou 4 du cycle hétérocyclique saturé,
Z représente le groupe méthylène et
$R^1$ et $R^2$ représentent des groupes alkyle ayant 1 à 4 atomes de carbone ou représentent avec l'atome d'azote inclus le reste 1-pyrrolidinyle, 1-pipéridinyle, 4-morpholinyle ou hexahydro-1H-1-azépinyle, leurs isomères optiquement actifs et leurs sels, compatibles du point de vue physiologique,
avec des acides inorganiques ou organiques.

**3.** 6,11-dihydro-11-[[3-[2-(1-pipéridinyl)éthyl]-1-pipéridinyl]carbonyl]-5H-pyrido[2,3-b][1,5]benzodiazépine-5-one et 5,11-dihydro-11-[[3-[3-(1-pipéridinyl)propyl]-1-pipéridinyl]carbonyl]-6H-pyrido[2,3-b][1,4]-benzodiazépine-6-one, leurs isomères optiquemen t actifs et leurs sels compatibles du point de vue physiologique avec des acides inorganiques ou organiques, selon la revendication 1.

**4.** 5,11-dihydro-11-[[4-(1-méthyl-4-pipéridinyl)-1-pipéridinyl]carbonyl]-6H-pyrido[2,3-b][1,4]benzodiazépine-6-one et 5,11-dihydro-11-[[2-[2-[[1-(phénylméthyl)-4-pipéridinyl]-méthylamino]éthyl]-1-pyrrolidinyl]-carbonyl]-6H-pyrido[2,3-b][1,4]benzodiazépine-6 -one, leurs isomères optiquement actifs et leurs sels, compatibles du point de vue physiologique, avec des acides inorganiques ou organiques.

**5.** Médicament contenant un ou plusieurs composés selon les revendications 1 à 3 ainsi que des véhicules et/ou adjuvants habituels.

**6.** Utilisation de composés selon les revendications 1 à 3 pour la préparation de médicaments pour le traitement des bradycardies et des bradyarythmies.

**7.** Procédé de préparation de diazépinones condensées de formule générale I selon la revendication 1 et de leurs isomères optiquement actifs et sels, compatibles du point de vue physiologique, avec des acides inorganiques ou organiques, caractérisé en ce que

a) pour la préparation de diazépinones condensées, substituées de manière basique, de formule générale Ia

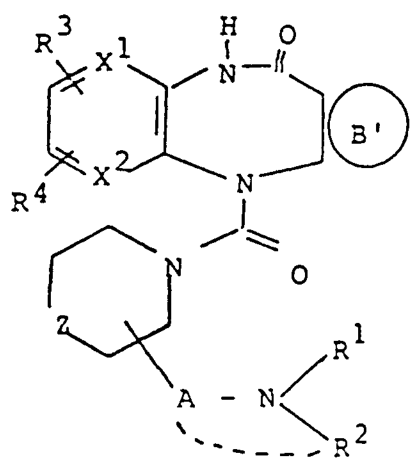

(Ia)

dans laquelle

$R^1$, $R^2$, $R^3$, $R^4$, $X^1$, $X^2$, A et Z ont les significations indiquées ci-dessus et

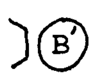

représente l'un des restes divalents (S), (U), (V), (W) ou (T')

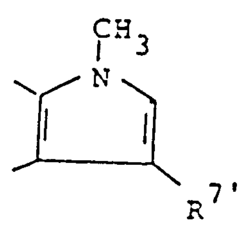

(T')

$R^{7'}$ étant un atome de chlore ou un groupe méthyle,

des dérivés d'acide carbonique de formule générale II

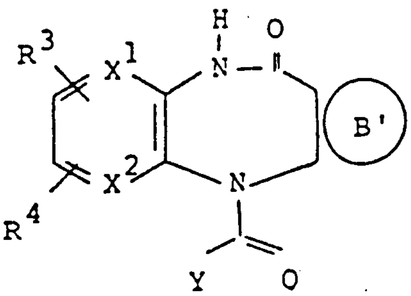

(II)

dans laquelle
$R^3$, $R^4$,

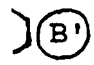 ,

$X^1$, $X^2$ ont les significations indiquées et Y représente un atome d'halogène, de préférence l'atome de brome ou de chlore, ou le reste $OR^{11}$, $R^{11}$ étant un reste alkyle éventuellement halogénosubstitué ayant 1 à 5 atomes de carbone, un reste phényle éventuellement substitué par des atomes d'halogène ou des groupes nitro ou un reste aralkyle ayant 7 à 15 atomes de carbone, sont mis à réagir avec des composés de formule générale III

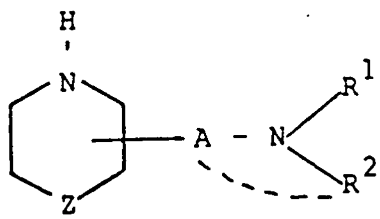

(III)

ou les composés métalliques correspondants de formule générale IIIa

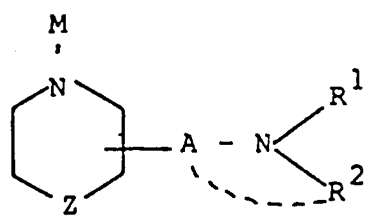

(IIIa)

dans les formules générales III et IIIa M représentant un atome de métal alcalin ou un équivalent d'un atome de métal alcalinoterreux et $R^1$, $R^2$, A et Z étant définis comme cidessus, à des températures situées entre -10 et +100 °C, éventuellement en présence de bases ou d'un excès d'un composé de formule générale III et d'un solvant, ou bien

b) pour la préparation de diazépinones condensées, substituées de manière basique, de formule générale Ia

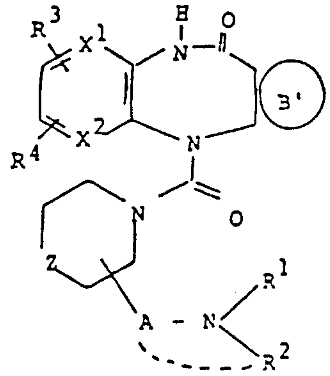

(Ia)

dans laquelle
$R^1$, $R^2$, $R^3$, $R^4$, $X^1$, $X^2$, A et Z ont les significations indiquées ci-dessus et

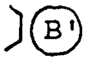

représente l'un des restes divalents (S), (U), (V), (W) ou (T')

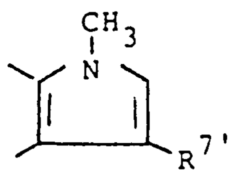

(T')

$R^{7'}$ étant un atome de chlore ou un groupe méthyle,
un composé tricyclique de formule générale IV

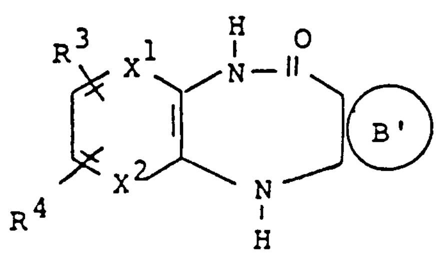

(IV)

dans laquelle les restes $R^3$, $R^4$, $X^1$, $X^2$ et

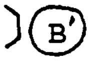

sont définis comme ci-dessus, est mis à réagir avec un dérivé d'acide chlorocarbonique de formule générale V

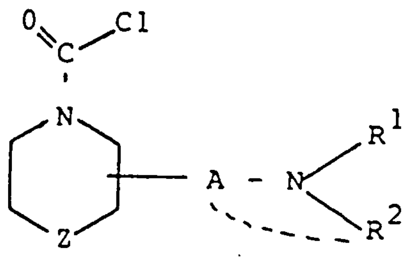

(V)

dans laquelle $R^1$, $R^2$, A et Z ont les significations indiquées ci-dessus, à des températures atteignant le point d'ébullition du mélange réactionnel, éventuellement en présence d'un solvant organique et/ou d'une base, ou bien

c) pour la préparation d'une diazépinone pyrrolo-condensée de formule générale Ib

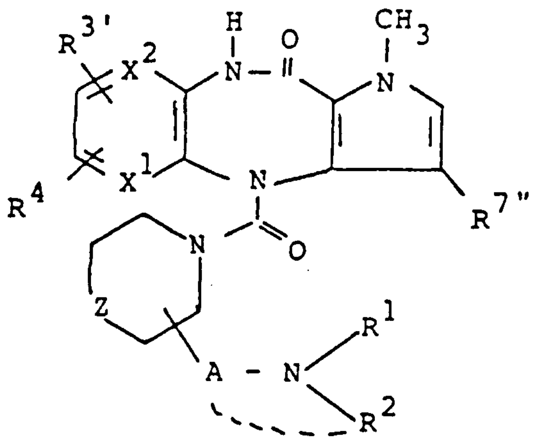

(Ib)

dans laquelle

$R^1$, $R^2$, $R^4$, $X^1$, $X^2$, A et Z ont les significations indiquées ci-dessus, pour Z à l'exception d'un atome de soufre, $R^{7}$" représentant un atome d'hydrogène et $R^{3'}$ un groupe alkyle ayant 1 à 4 atomes de carbone ou un atome d'hydrogène, un composé de formule générale Ib dans laquelle $R^7$ représente un atome de chlore est hydrogénolysé avec l'hydrogène en présence de catalyseurs et de solvants à des températures comprises entre 0 et 130 ° C et des pressions d'hydrogène de 1 à 300 bar ou avec l'acide formique en présence de solvants et de palladium ou d'acétate de palladium comme catalyseur à des températures situées entre 40 et 110 ° C,

et éventuellement les composés obtenus de formule générale I sont convertis en leurs sels d'addition d'acides avec des acides inorganiques ou organiques, ou bien les sels d'addition d'acides obtenus sont convertis en les bases libres et/ou les racémates obtenus sont résolus si on le souhaite en leurs isomères à l'aide d'acides optiquement actifs.

**8.** Procédé selon la revendication 7a, caractérisé en ce que la réaction est accomplie en présence de solvants aprotiques polaires à des températures situées entre 40 et 100 ° C en présence d'hydroxydes, alcoolates ou carbonates alcalins ou alcalinoterreux ou d'amines tertiaires.

**9.** Procédé selon la revendication 7b, caractérisé en ce que la réaction est accomplie en présence d'hydrocarbures aromatiques, dans des éthers, des cétones, des hydrocarbures aliphatiques chlorés, l'acétonitrile ou le diméthylformamide ou dans des mélanges de ces solvants et en présence d'amines tertiaires à des températures situées entre 30 et 100 ° C.

**10.** Procédé selon la revendication 7c, caractérisé en ce que l'hydrogénolyse est accomplie en présence de métaux du sous-groupe VIII du système périodique des éléments, éventuellement en présence d'accepteurs d'acide chlorhydrique ou à l'aide d'acide formique en présence de diméthylformamide et de palladium sur charbon ou à l'aide de formiate de triéthylammonium en présence d'un excès de triéthylamine et de palladium sur noir animal ou d'acétate de palladium et de triarylphosphines à des

températures situées entre 40 et 110 ° C.

**Revendications pour les Etats contractants suivants : ES, GR**

**1.** Procédé de préparation de diazépinones condensées de formule générale I

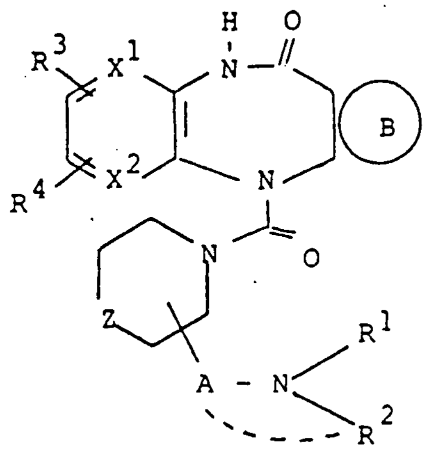

(I)

dans laquelle

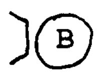

représente l'un des restes divalents

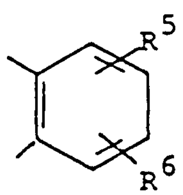

,

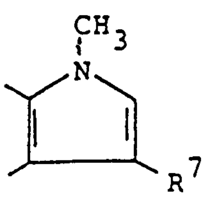

,

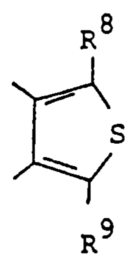

,

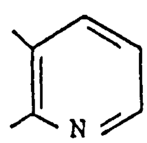

ou

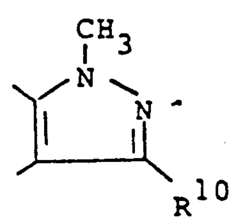

(S) (T) (U) (V) (W)

et

$X^1$, $X^2$, A, $R^1$ à $R^{10}$ et Z ont les significations suivantes:

$X^1$ et $X^2$ représentent un groupe =CH ou, lorsque

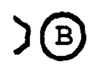

revêt les significations des restes divalents (S), (U) ou (W) cités ci-dessus, peuvent aussi représenter tous les deux ou seulement $X^1$ ou seulement $X^2$ un atome d'azote;

A est un reste alkylène saturé linéaire ou ramifié ayant deux à sept atomes de carbone;

qui peut aussi être interrompu par un atome d'oxygène ou de soufre ou par le groupe méthylimino ou éthylimino;

Z est une liaison simple, un atome d'oxygène ou de soufre, le groupe méthylène ou 1,2-éthylène;

$R^1$ est un reste alkyle ramifié ou non ramifié ayant 1 à 4 atomes de carbone ou le groupe benzyle;

$R^2$ est un reste alkyle ramifié ou non ramifié ayant 1 à 7 atomes de carbone qui peut encore être substitué éventuellement sur son deuxième à septième atome de carbone par un groupe hydroxyle, un

reste cycloalkyle ou cycloalkylméthyle ayant 3 à 7 atomes de carbone dans le cycle, la cycle cycloalkyle pouvant éventuellement être substitué encore par un groupe hydroxyle;
$R^1$ et $R^2$ peuvent cependant former aussi avec l'atome d'azote situé entre eux un cycle hétérocyclique saturé, monocyclique, ayant 4 à 7 chaînons qui peut éventuellement être interrompu par un atome d'oxygène ou par le groupe N-$CH_3$;
$R^2$ peut aussi être relié à A par un pont alkylène pour former avec le groupe $NR^1$ un système cyclique hétérocyclique saturé à 5, 6 ou 7 chaînons;
$R^3$ est un groupe alkyle ayant 1 à 4 atomes de carbone, un atome de chlore ou un atome d'hydrogène;
$R^4$ est un atome d'hydrogène ou un groupe méthyle;
$R^5$ et $R^6$ représentent chacun un atome d'hydrogène, un atome de fluor, de chlore ou de brome au un groupe alkyle ayant 1 à 4 atomes de carbone;
$R^7$ est un atome d'hydrogène ou de chlore ou un groupe méthyle;
$R^8$ est un atome d'hydrogène ou un reste alkyle ayant 1 à 4 atomes de carbone;
$R^9$ est un atome d'hydrogène ou un atome d'halogène ou un groupe alkyle ayant 1 à 4 atomes de carbone; et
$R^{10}$ est un atome d'hydrogène ou un groupe méthyle, à condition que lorsque

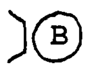

représente le reste divalent (T) et $R^7$ est un atome d'hydrogène, $R^3$ ait les significations indiquées ci-dessus à l'exception d'un atome de chlore et Z ait les significations indiquées ci-dessus à l'exception d'un atome de soufre, leurs isomères optiquement actifs et leurs sels, compatibles du point de vue physiologique, avec des acides inorganiques et organiques, caractérisé en ce que

a) pour la préparation de diazépinones condensées, substituées de manière basique, de formule générale Ia

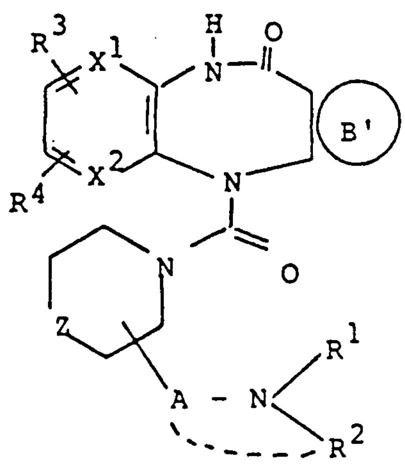

(Ia)

dans laquelle
$R^1$, $R^2$, $R^3$, $R^4$, $X^1$, $X^2$, A et Z ont les significations indiquées ci-dessus et

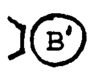

représente l'un des restes divalents (S), (U), (V), (W) ou (T')

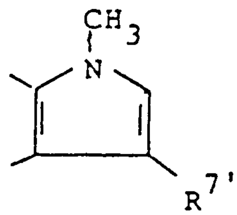

(T')

$R^{7'}$ étant un atome de chlore ou un groupe méthyle, des dérivés d'acide carbonique de formule générale II

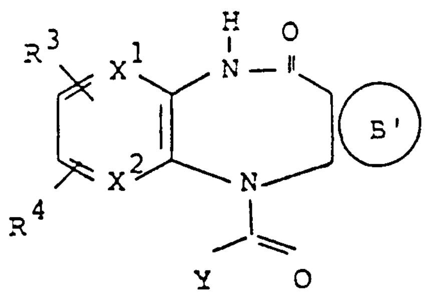

(II)

dans laquelle
$R^3$, $R^4$,

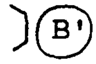 ,

$X^1$, $X^2$ ont les significations indiquées et Y représente un atome d'halogène, de préférence l'atome de brome ou de chlore, ou le reste $OR^{11}$, $R^{11}$ étant un reste alkyle éventuellement halogénosubstitué ayant 1 à 5 atomes de carbone, un reste phényle éventuellement substitué par des atomes d'halogène ou des groupes nitro ou un reste aralkyle ayant 7 à 15 atomes de carbone, sont mis à réagir avec des composés de formule générale III

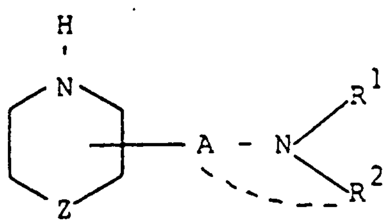

(III)

ou les composés métalliques correspondants de formule générale IIIa

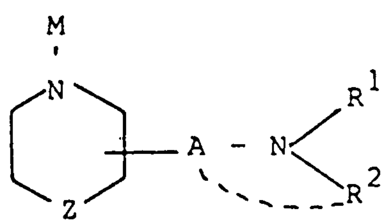

(IIIa)

dans les formules générales III et IIIa M représentant un atome de métal alcalin ou un équivalent d'un atome de métal alcalinoterreux et $R^1$, $R^2$, A et Z étant définis comme cidessus, à des températures situées entre -10 et +100 °C, éventuellement en présence de bases ou d'un excès

d'un composé de formule générale III et d'un solvant, ou bien

b) pour la préparation de diazépinones condensées, substituées de manière basique, de formule générale Ia

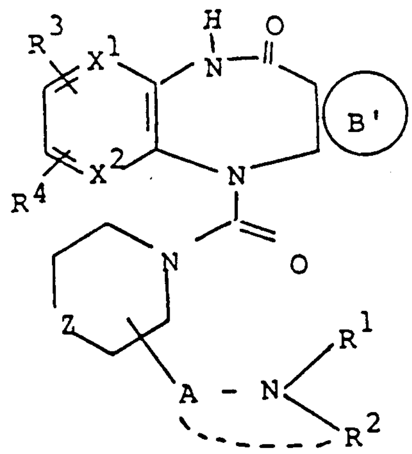

(Ia)

dans laquelle

$R^1$, $R^2$, $R^3$, $R^4$, $X^1$, $X^2$, A et Z ont les significations indiquées ci-dessus et

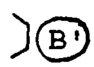

représente l'un des restes divalents (S), (U), (V), (W) ou (T')

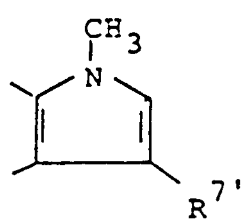

(T')

$R^{7'}$ étant un atome de chlore ou un groupe méthyle,

un composé tricyclique de formule générale IV

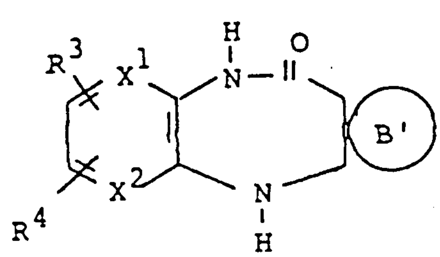

(IV)

dans laquelle les restes $R^3$, $R^4$, $X^1$, $X^2$ et

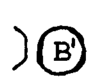

sont définis comme ci-dessus, est mis à réagir avec un dérivé d'acide chlorocarbonique de formule

générale V

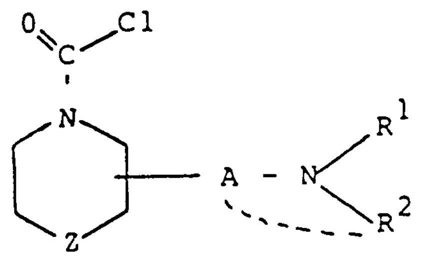

(V)

dans laquelle $R^1$, $R^2$, A et Z ont les significations indiquées ci-dessus, à des températures atteignant le point d'ébullition du mélange réactionnel, éventuellement en présence d'un solvant organique et/ou d'une base, ou bien

c) pour la préparation d'une diazépinone pyrrolo-condensée de formule générale Ib

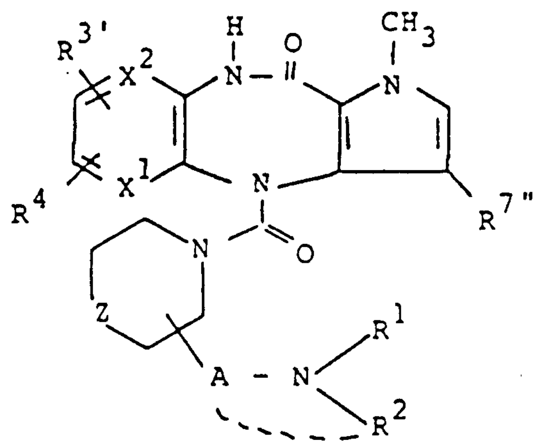

(Ib)

dans laquelle

$R^1$, $R^2$, $R^4$, $X^1$, $X^2$, A et Z ont les significations indiquées ci-dessus, pour Z à l'exception d'un atome de soufre, $R^{7}$" représentant un atome d'hydrogène et $R^{3}$' un groupe alkyle ayant 1 à 4 atomes de carbone ou un atome d'hydrogène, un composé de formule générale Ib dans laquelle $R^7$ représente un atome de chlore est hydrogénolysé avec l'hydrogène en présence de catalyseurs et de solvants à des températures comprises entre 0 et 130 °C et des pressions d'hydrogène de 1 à 300 bar ou avec l'acide formique en présence de solvants et de palladium ou d'acétate de palladium comme catalyseur à des températures situées entre 40 et 110 °C,

et éventuellement les composés obtenus de formule générale I sont convertis en leurs sels d'addition d'acides avec des acides inorganiques ou organiques, ou bien les sels d'addition d'acides obtenus sont convertis en les bases libres et/ou les racémates obtenus sont résolus si on le souhaite en leurs isomères à l'aide d'acides optiquement actifs.

2. Procédé selon la revendication 1a, caractérisé en ce que la réaction est accomplie en présence de solvants aprotiques polaires à des températures situées entre 40 et 100 °C en présence d'hydroxydes, alcoolates ou carbonates alcalins ou alcalinoterreux ou d'amines tertiaires.

3. Procédé selon la revendication 1b, caractérisé en ce que la réaction est accomplie en présence d'hydrocarbures aromatiques, dans des éthers, des cétones, des hydrocarbures aliphatiques chlorés, l'acétonitrile ou le diméthylformamide ou dans des mélanges de ces solvants et en présence d'amines tertiaires à des températures situées entre 30 et 100 °C.

4. Procédé selon la revendication 1c, caractérisé en ce que l'hydrogénolyse est accomplie en présence de métaux du sous-groupe VIII du système périodique des éléments, éventuellement en présence

d'accepteurs d'acide chlorhydrique ou à l'aide d'acide formique en présence de diméthylformamlde et de palladium sur charbon ou à l'aide de formiate de triéthylammonium en présence d'un excès de triéthylamine et de palladium sur noir animal ou d'acétate de palladium et de triarylphosphines à des températures situées entre 40 et 110 ° C.